# EUROPEAN PATENT APPLICATION

(11) **EP 4 653 044 A2**
(43) Date of publication of application: **26.11.2025**
(21) Application number: 25207303.6
(22) Date of filing: 17.11.2022
(51) Int. Cl.: A61N 1/36

(54) **METHOD AND SYSTEM FOR EYE TREATMENT**

(30) Priority: 19.11.2021 US 202163281558 P
(62) Divisional of application: 22896495.3
(71) Applicant: i-Lumen Scientific, Inc., Bloomington, MN 55431 (US)
(72) Inventor: MASKO, Marshall T., Bloomington, 55431 (US); VELURE, John C., Bloomington, 55431 (US); MINOGUE, Conor M., Galway (IE)
(74) Representative: Murgitroyd & Company

(57) **Abstract**

Methods and systems including a first electrode substrate having one or more stimulation electrodes that are configured to apply electrical stimulation signals to a patient's first eye; a plurality of sensor electrodes configured to detect electrical activity from retinal or neural cells of the patient during delivery of an electrical-stimulation therapy, wherein the plurality of sensor electrodes includes a first sensor electrode and a second sensor electrode; a first return electrode configured to be placed in a location on the patient such that an electrical current is formed between at least one of the one or more stimulation electrodes and the first return electrode; and a stimulation controller configured to control the electrical current formed between the at least one of the one or more stimulation electrodes and the first return electrode such that the electrical current passes through a retina of the patient's first eye and delivers the electrical-stimulation therapy.

## Description

### CROSS-REFERENCE TO RELATED APPLICATIONS

This application claims priority benefit, including under 35 U.S.C. §119(e), of U.S. Provisional Patent Application No. 63/281,558, filed November 19, 2021 by Marshall T. Masko et al., titled "METHOD AND SYSTEM FOR EYE TREATMENT," which is incorporated herein by reference in its entirety.

This application is related to:
U.S. Patent 10,391,312, issued on August 27, 2019 by Mowery et al., titled "APPARATUS AND METHOD FOR OCULAR MICROCURRENT STIMULATION THERAPY", PCT Patent Application PCT/US2016/051550 (published as WO 2017/048731), filed September 13, 2016 by Mowery et al., titled "APPARATUS AND METHOD FOR OCULAR MICROCURRENT STIMULATION THERAPY",
U.S. Provisional Patent Application 62/283,870, filed September 15, 2015 by Mowery et al., titled "APPLIANCE FOR MICROCURRENT STIMULATION THERAPY USING A DISPOSABLE MATERIAL AFIXED TO THE UPPER AND LOWER EYE LID & OTHER BODY PARTS",
U.S. Provisional Patent Application 62/283,871, filed September 15, 2015 by Masko et al., titled "APPARATUS FOR A METHOD OF APPLICATION OF MICROCURRENT STIMULATION THERAPY, CONSISTING OF A GOGGLE DEVICE AFFIXED TO & ENCIRCLING THE UPPER AND/OR LOWER EYELIDS, AS WELL AS OTHER BODY PARTS",
U.S. Provisional Patent Application 62/365,838, filed July 22, 2016 by Tapp et al., titled "APPLIANCE FOR MICRO-CURRENT STIMULATION",
U.S. Patent Application 17/415,508 (published as US 2022/0047866), filed June 17, 2021 by Masko et al., titled "APPARATUS AND METHOD FOR MICROCURRENT STIMULATION THERAPY",
U.S. Patent Application 17/416,024 (published as US 2022/0062634), filed June 18, 2021 by Masko et al., titled "MICROCURRENT-STIMULATION-THERAPY APPARATUS AND METHOD",
PCT Patent Application PCT/US2019/063404 (published as WO 2020/131329), filed on November 26, 2019, by Masko et al., titled "APPARATUS AND METHOD FOR MICROCURRENT STIMULATION THERAPY",
PCT Application PCT/US2019/067627 (published as WO 2020/132337), filed on December 19, 2019 by Masko et al., titled "MICROCURRENT-STIMULATION-THERAPY APPARATUS AND METHOD",
U.S. Provisional Patent Application 62/783,116 filed on December 20, 2018 by Masko et al., titled "APPARATUS AND METHOD FOR MICROCURRENT STIMULATION THERAPY", PCT Patent Application PCT/US2020/021267 (published as WO 2021/177968), filed March 5, 2020 by Mowery et al. titled "VISION TESTING AND TREATMENT SYSTEM AND METHOD",
PCT Patent Application PCT/US2021/031869 (published as WO 2021/231496), filed May 11, 2021 by Duncan et al. titled "ELECTRODE SYSTEM FOR VISION TREATMENT AND METHOD",
U.S. Provisional Patent Application 63/025,987 filed on May 15, 2020 by Duncan et al., titled "ELECTRODE SYSTEM FOR VISION TREATMENT AND METHOD", and
U.S. Patent 11,116,973, issued on September 14, 2021 by Masko et al., titled "SYSTEM AND METHOD FOR A MEDICAL DEVICE",
each of which is incorporated herein by reference in its entirety.

### FIELD OF THE INVENTION

The present invention relates generally to electrical stimulation of the human body, and in particular, to methods and systems for applying electrical stimulation to the eye to treat various eye conditions such as retinal diseases including Macular Degeneration (dry or wet AMD (Age-related Macular Degeneration)), Retinitis Pigmentosa, Diabetic Macular Edema, Retinal Tear or Detachment, Macular Hole, and Epiretinal Membrane.

### BACKGROUND OF THE INVENTION

There is increasing interest in the use of non-invasive electrical stimulation of the eye to treat various conditions. The basis of the therapeutic strategy may include stimulation of neurons along the visual pathway and/or non-neural tissue such as glial cells and epithelial cells. The principal noninvasive routes by which electrical stimulation has been delivered to patients with the purpose of improving vision are transpalpebral, transorbital, and transcorneal. Transpalpebral electrical stimulation uses a small surface electrode placed on the eyelid with a return electrode that can be located on the back of the head, on the neck or shoulder, on the side of the head, or on any other suitable location on the patient that provides a return pathway for the electrical stimulation.

Electrical stimulation is usually characterized by a threshold effect; that is, the current density at the target tissue has to be high enough, and be sustained for long enough, to activate the specific neural or non-neural cells involved. The current delivered at the eyelid, or at the cornea, disperses through and around the eye and then further throughout the head before it concentrates again at the return electrode. The current density at any point in the eye or head depends on the relative electrical conductivities of the different types of tissue involved, such as bone, fat, ocular media, neural tissue etc. The current density at any point can be estimated by computational modelling, but in practice it is hard to select the current amplitude at the eyelid or cornea that will reach the desired threshold at the target tissue.

U.S. Patent 5,154,174 by Marko Hawlina, issued on October 13, 1992 with the title "Electrode for electroretinography and method of use," and is incorporated herein by reference. U.S. Patent 5,154,174 describes an electrode for electroretinography that comprises a plastically deformable, self-supporting, filamentary electrically conductive member having first and second electrically insulated zones between which a portion of the member is exposed as an electrical contact. The member preferably forms an endless loop.

U.S. Patent 5,522,864 by Larry B. Wallace et al., issued on June 4, 1996 with the title "Apparatus and method for ocular treatment," and is incorporated herein by reference. U.S. Patent 5,522,864 describes that macular degeneration and other ocular pathology in a subject are treated by the steps of: placing a positive electrode of a direct current source in electrical contact with a closed eyelid of a subject; placing a negative electrode of the source in electrical contact with the posterior neck of the subject; and causing a constant direct current of 200 µA to flow between the electrodes through the subject for about 10 minutes. The source can be a portable, battery powered constant direct current generator which is affixed to the subject. The subject can ambulate during treatment.

U.S. Patent 6,035,236 by John B. Jarding, et al., issued on March 7, 2000 with the title "Methods and apparatus for electrical microcurrent stimulation therapy" and is incorporated herein by reference in its entirety. Patent 6,035,236 describes an apparatus for supplying an electrical signal to a body part in order to provide microcurrent stimulation therapy to the body part. The apparatus preferably includes a first sweep wave or sweep frequency signal generator configured to generate a first sweep wave signal, a buffer amplifier circuit configured to receive the first sweep wave signal from the first sweep signal generator and amplify and buffer the sweep wave signal creating a buffered sweep wave signal. In addition, the apparatus preferably includes a current limiting circuit configured to receive the buffered sweep wave signal from the buffer amplifier circuit and limit the amount of current supplied to the body part. Finally, the apparatus preferably comprises a probe for applying the sweep wave signal to the body part. The apparatus may further comprise a second signal generator for generating a second signal which may comprise either a sweep wave signal or a non-sweep wave signal. The apparatus also will include a signal combining circuit configured to receive the first and second signals from the first and second signal generators and combine the first and second signals into a composite sweep wave signal.

U.S. Patent 6,275,735 by John B. Jarding et al., issued on August 14, 2001 with the title "Methods and apparatus for electrical microcurrent stimulation therapy" and is incorporated herein by reference in its entirety. Patent 6, 275,735 describes a method and apparatus for providing microcurrent stimulation therapy to a body part. In one embodiment, a method allows digital control of the modulation frequency of the microcurrent signal. The method includes receiving a first digital data word which is used to produce a first frequency related to the first digital data word, whereupon, a first microcurrent signal at the first frequency is applied to the body part. A second digital data word is received and used to produce a second frequency related to the second digital data word. A second microcurrent signal at the second frequency is applied to the body part. In another embodiment, a method allows direct digital synthesis of the microcurrent stimulation signal. A first digital data word is used to produce a first analog voltage which is applied to the body part. A second digital data word is used to produce a second analog voltage which is also applied to the body part, where the first analog voltage is different from the second analog voltage. In yet another embodiment, an apparatus for providing microcurrent stimulation therapy includes a digital-to-analog converter, a controller and a plurality of data words. The controller is coupled to the digital-to-analog converter and supplies the digital-to-analog converter with digital data words in order to generate an electrical signal for the microcurrent stimulation therapy.

United States Patent 7,062,319 by Jouni Ihme, et al., issued on June 13, 2006 with the title "Method and arrangement for determining suitable treatment frequency and/or intensity," and is incorporated herein by reference. Patent 7,062,319 describes a method and arrangement for determining a suitable treatment frequency and/or intensity of a treatment signal used in electrical treatment. In the method, a stimulating electrical signal is directed to an object to produce different reaction types in the object at different intensities of the stimulating electrical signal. For at least three different reaction types, the intensity of the stimulating electrical signal at which a reaction type occurred is stored. The electrical signal intensities stored for the different reaction types at least at three different frequencies are compared with reference values and the frequency and/or signal intensity at which the signal intensity deviates sufficiently from one or more reference values is determined. The method utilizes the frequency and/or signal intensity found in the process in determining the suitable treatment frequency and/or signal intensity.

United States Patent 7,158,834 by Edward L. Paul, Jr., issued on January 2, 2007 with the title "Method and apparatus for performing microcurrent stimulation (MSC) therapy," and is incorporated herein by reference. Patent 7,158,834 describes a method and apparatus for providing microcurrent stimulation (MSC) therapy. Patent 7,158,834 states: it has been determined that the application of microcurrent signals at particular frequencies to the eye for particular periods of time stabilizes and even improves conditions of macular degeneration and other ocular diseases.

United States Patent 7,326,181 by Jefferson J. Katims, issued on February 5, 2008 with the title "Nervous tissue stimulation device and method," and is incorporated herein by reference. Patent 7,326,181 describes a method using a precisely controlled, computer programmable stimulus for neuroselective tissue stimulation that does not leave a sufficient voltage or electrical artifact on the tissue being stimulated that would interfere or prevent a monitoring system from recording the physiological response is utilized to evaluate the physiological conduction of the tissue being studied. A computer controls both the waveform, duration and intensity of the stimulus. An output trigger to the nerve response recording component controls the timing of its operation. A neuroselective nervous tissue response latency and amplitude may be determined. The computer controlled stimulus may also be administered for therapeutic purposes.

United States Patent 8,843,209 by Paul W. Wacnik et al., issued on September 23, 2014 with the title "Ramping parameter values for electrical stimulation therapy," and is incorporated herein by reference. Patent 8,843,209 describes devices, systems, and techniques for ramping one or more parameter values of electrical stimulation. An implantable medical device may increase or decrease a parameter value, e.g., amplitude or pulse width, over time to reach a target value of the parameter. In one example, a memory may be configured to store a plurality of amplitude ramp schedules. At least one processor may be configured to obtain a stimulation parameter set that at least partially defines an electrical stimulation therapy, select one of the plurality of amplitude ramp schedules based on a signal frequency of the stimulation parameter set, and increase an amplitude of the electrical stimulation therapy during a ramp period defined by the selected amplitude ramp schedule.

United States Patent 9,199,080 by Florian Gekeler et al., issued on December 1, 2015 with the title "Method for treating an eye," and is incorporated herein by reference. According to Patent 9,199,080, in a method for treating at least one eye of a patient in need of such treatment with a pulsed electrical stimulation signal, first an individual parameter of the patient is determined, thereafter at least one stimulation parameter of the pulsed electrical stimulation signal is set depending on the at least one individual parameter, and then the pulsed electrical stimulation signal is applied to the at least one eye.

United States Patent 9,724,230 by Paul Badawi, issued on August 8, 2017 with the title "Dry eye treatment apparatus and methods," and is incorporated herein by reference. Patent 9,724,230 describes dry eye treatment apparatus and methods which generally include a patch or strip affixed to the skin of the upper and/or lower eyelids to deliver heat or other forms of energy, pressure, drugs, moisture, etc. (alone or in combination) to the one or more meibomian glands contained within the underlying skin. The treatment strip or strips include one or more strips configured to adhere to an underlying region of skin in proximity to one or both eyes of a subject such that the one or more strips allow for the subject to blink naturally without restriction from the one or more strips. Moreover, the one or more strips may be configured to emit energy to the underlying region of skin and where the one or more strips are shaped to follow a location of one or more meibomian glands contained within the underlying region of skin.

Publication titled "Evaluation of residual retinal function by pupillary constrictions and phosphenes using transcorneal electrical stimulation in patients with retinal degeneration," by Takeshi Morimoto et al., Graefe's Archive for Clinical and Experimental Ophthalmology, Vol. 244, pp. 1283-1292 (2006) is incorporated herein by reference.

Publication titled "Effect of transcorneal electrical stimulation in patients with nonarteritic ischemic optic neuropathy or traumatic optic neuropathy," by Takashi Fujikado et al., Japanese Journal of Ophthalmology, Vol. 50, pp. 266-273 (2006) is incorporated herein by reference.

Publication titled "Measuring pupil size and light response through closed eyelids," by Yousef Farraj et al., Biomedical Optics Express, Vol. 12, No. 10, pp. 6485-6495 (2021) is incorporated herein by reference.

There is a need for an improved method and system for treating eye conditions using electrical stimulation.

### SUMMARY OF THE INVENTION

In some embodiments, the present invention provides a method and system for providing electrical stimulation of the eye (in some such embodiments, the electric current used in the electrical stimulation of the eye takes the form of low frequency (less than about 1000 Hz) pulse trains where individual pulses may be monophasic (of either polarity) or biphasic). As used herein, the "pulse-repetition rate" or "pulse rate" of a pulse train is referred to informally as "frequency" and/or "Hz," and references to numerical values for frequency and/or Hz are to be understood as values for the number of pulses per second in a pulse train. In some embodiments, the electrical stimulation includes the forming of pulse trains comprising sequences of sub-trains where the pulses in each sub-train are of the same type and polarity. In some embodiments, the pulses are current controlled, meaning that the drive circuitry is designed to deliver the selected current irrespective of the circuit impedance, or voltage controlled, where the current depends on the circuit impedance.

In some embodiments, the patient report of phosphenes is used as an indicator that the electrical stimulus is reaching the target retinal tissues. As used herein, "phosphenes" are impressions of light (e.g., visual flashes) within the eye that occur without light entering the eye that are caused by stimulation of the retina (e.g., stimulation of specific cells within the retina such as bipolar cells). In some embodiments, the efficacy in treating retinal diseases (e.g., macular degeneration) to restore or maintain vision is integrally linked to delivering a level of ocular stimulation (e.g., electrical stimulation) that induces the visible appearance of phosphenes when a patient's eyes are closed.

In some embodiments, the present invention provides an objective means to monitor the response of the eye during therapy such that a therapeutic stimulation intensity is achieved, and such that excessive stimulation is avoided. In some such embodiments, the present invention recovers electrical signals in the region of the eye and head during electrical stimulation in order to observe the electrical activity of neural cells in the retina, optic nerve, and/or the brain in response to the electrical stimulation. In some embodiments, the electrical-stimulation responses occur in the period after delivery of the stimulation pulse and are extremely low-level signals embedded in noise. In some embodiments, in order to extract the electrical-stimulation-response signals, waveform records from multiple sequential identical pulses are digitally captured and the resulting waveforms are averaged at each timepoint such that random noise cancels out while the underlying signal emerges.

In some embodiments, the present invention provides a system for performing noninvasive electrical stimulation of the eye that includes a first electrode located at the front of the eye either directly on the cornea or on the overlying eyelid, a second return electrode located posteriorly from the eye on the back of the head or on side of the head or on the neck or shoulder, a pulsed current generator which passes a therapeutic electrical current between the first and second electrodes, a third sensing electrode located on the head together with a corresponding reference electrode, a differential amplifier which measures the potential difference between the third electrode and the reference electrode, and a controller which is configured to deliver the electrical stimulus to the eye and measure the subsequent electrical response of the eye.

In some embodiments, the present invention provides a system that includes a first electrode substrate that includes: one or more electrodes on the first electrode substrate that are configured to apply electrical-stimulation therapy to a first eye of a patient, the first eye having an upper eyelid and a lower eyelid, wherein the first electrode substrate is configured to locate the one or more electrodes on an outer surface of at least one of the upper eyelid and the lower eyelid of the patient's first eye, a stimulation controller configured to control the electrical-stimulation therapy, wherein each of the one or more electrodes is operatively coupled to the stimulation controller, wherein the stimulation controller is further configured to: set a current level of the electrical-stimulation therapy at a first value during a first treatment session, wherein the first value of the current level is below a stimulation threshold of the first eye, increase the current level from the first value during the first treatment session, and receive an indication during the first treatment session that phosphenes are visible to the patient in the first eye, wherein the increase of the current level from the first value is stopped at a second value when the indication is received.

In some embodiments, the present invention provides a method that includes providing a first electrode substrate, wherein the first electrode substrate includes: one or more electrodes on the first electrode substrate that are configured to apply electrical-stimulation therapy to a first eye of a patient, the first eye having an upper eyelid and a lower eyelid, wherein the first electrode substrate is configured to locate the one or more electrodes on an outer surface of at least one of the upper eyelid and the lower eyelid of the patient's first eye, attaching the first electrode substrate to the patient's skin such that the one or more electrodes are on the outer surface of at least one of the upper eyelid and the lower eyelid of the patient's first eye; applying the electrical-stimulation therapy to the first eye during a first treatment session, wherein the applying of the therapy to the first eye during the first treatment session includes: setting a current level of the therapy applied to the first eye at a first value, wherein the first value of the current level is below a stimulation threshold of the first eye, increasing the current level from the first value, and receiving an indication that phosphenes are visible to the patient in the first eye, wherein the increasing of the current level from the first value is stopped at a second value when the indication is received.

In some embodiments, the present invention provides a non-transitory computer-readable medium having instructions stored thereon for causing a suitably programmed control system to execute a method of providing an electrical-stimulation therapy to a first eye of a patient, the first eye having an upper eyelid and a lower eyelid, wherein the method is executed by the control system on an electrical-stimulation system that includes one or more electrodes, wherein the one or more electrodes are on a first electrode substrate, wherein the first electrode substrate is attached to the patient's skin such that the one or more electrodes are on the outer surface at least one of the upper eyelid and the lower eyelid of the patient's first eye, the method including applying the electrical-stimulation therapy to the first eye during a first treatment session, wherein the applying of the therapy to the first eye during the first treatment session includes: setting a current level of the therapy at a first value, wherein the first value of the current level is below a stimulation threshold of the first eye, increasing the current level from the first value, and receiving an indication that phosphenes are visible to the patient in the first eye, wherein the increasing of the current level from the first value is stopped at a second value when the indication is received.

### BRIEF DESCRIPTION OF THE DRAWINGS

FIG. 1A is a perspective-view diagram of an eye-stimulation system 101 that uses an eye-stimulation electrode system 150, according to some embodiments of the present invention.
FIG. 1B is a perspective-view diagram of an eye-stimulation system 102 that uses an eye-stimulation electrode system 151, according to some embodiments of the present invention.
FIG. 1C is a perspective-view diagram of an eye-stimulation system 103, according to some embodiments of the present invention.
FIG. 2A is a block diagram/flow chart of an eye-stimulation system and method 201 for electrical stimulation applied to human eyes, according to some embodiments of the present invention.
FIG. 2B is a block diagram/flow chart of an eye-stimulation system and method 202 for electrical stimulation applied to human eyes, according to some embodiments of the present invention.
FIG. 3A is a graph 301 of electrical signal amplitude over time as applied to an eye of a patient during an initial sub-session 350 used to determine an amplitude of signal that is both effective and not too uncomfortable to the patient, according to some embodiments of the present invention.
FIG. 3B is a block diagram of a system 303 that can vary amplitude and/or waveform of signals over time as applied to an eye of a patient, such that the amplitude and/or waveform of the signals is both effective and not too uncomfortable to the patient according to some embodiments of the present invention.
FIG. 4 is an example electroretinogram (ERG) 401 generated in response to a light flash.
FIG. 5 is a graph 501 showing individual components of a visually evoked response (VER).
FIG. 6A is a schematic front-view diagram of an electrical stimulation system 601 positioned on patient 99, according to some embodiments of the present invention.
FIG. 6B is a schematic front-view diagram of an electrical stimulation system 602 positioned on patient 99, according to some embodiments of the present invention.
FIG. 6C is a schematic front-view diagram of an electrical stimulation system 603 positioned on patient 99, according to some embodiments of the present invention.
FIG. 6D is a schematic side-view diagram of an electrical stimulation system 604 positioned on patient 99, according to some embodiments of the present invention.
FIG. 7 is a schematic front-view diagram of an electrical stimulation system 701 positioned on patient 99, according to some embodiments of the present invention.

### DESCRIPTION OF PREFERRED EMBODIMENTS

Although the following detailed description contains many specifics for the purpose of illustration, a person of ordinary skill in the art will appreciate that many variations and alterations to the following details are within the scope of the invention. Specific examples are used to illustrate particular embodiments; however, the invention described in the claims is not intended to be limited to only these examples, but rather includes the full scope of the attached claims. Accordingly, the following preferred embodiments of the invention are set forth without any loss of generality to, and without imposing limitations upon the claimed invention. Further, in the following detailed description of the preferred embodiments, reference is made to the accompanying drawings that form a part hereof, and in which are shown by way of illustration specific embodiments in which the invention may be practiced. It is understood that other embodiments may be utilized and structural changes may be made without departing from the scope of the present invention.

It is specifically contemplated that the present invention includes embodiments having combinations and subcombinations of the various embodiments and features that are individually described herein (i.e., rather than listing every combinatorial of the elements, this specification includes descriptions of representative embodiments and contemplates embodiments that include some of the features from one embodiment combined with some of the features of another embodiment, including embodiments that include some of the features from one embodiment combined with some of the features of embodiments described in the patents and application publications incorporated by reference in the present application). Further, some embodiments include fewer than all the components described as part of any one of the embodiments described herein.

The leading digit(s) of reference numbers appearing in the Figures generally corresponds to the Figure number in which that component is first introduced, such that the same reference number is used throughout to refer to an identical component which appears in multiple Figures. Signals and connections may be referred to by the same reference number or label, and the actual meaning will be clear from its use in the context of the description.

Certain marks referenced herein may be common-law or registered trademarks of third parties affiliated or unaffiliated with the applicant or the assignee. Use of these marks is for providing an enabling disclosure by way of example and shall not be construed to limit the scope of the claimed subject matter to material associated with such marks.

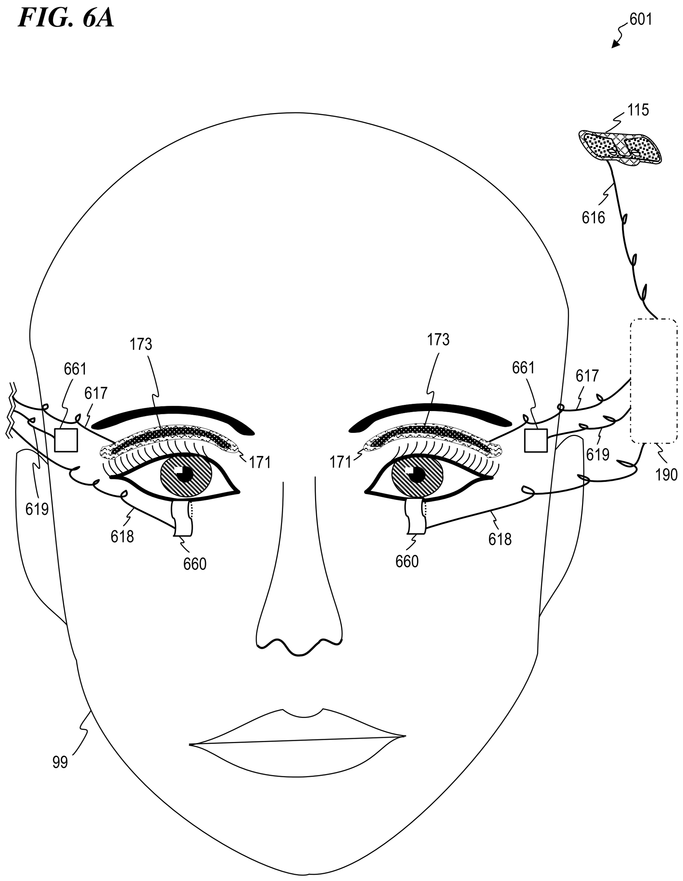

In some embodiments, the present invention uses patient-reported sighting of phosphenes flashing in the eye (or objectively determined indications of phosphenes such as the configurations shown in Figures 6A-7 below) to set the electrical stimulation level for treating retinal diseases. In some embodiments, the initial indication of phosphenes is the threshold for the start of the low treatment setting. In some embodiments, the treatment range of electrical stimulation is increased (i.e., ramped up) via increasing the current (e.g., microamps) of the electrical stimulation as the patient continues to see phosphenes (in some embodiments, instead of (or in addition to) increasing the stimulation intensity by increasing the current, stimulation intensity is increased by increasing pulse width; in other embodiments, treatment intensity is controlled by controlling the voltage delivered across the stimulation electrodes, and in some such embodiments, pulse voltage and/or duration of the voltage pulse is incremented until the phosphene threshold is reached). In some embodiments, as the stimulation level delivered to the eye is increased, the threshold of when phosphenes are first visible is linked to the starting point of stimulation therapy for that, and potentially for subsequent, treatment sessions. In some embodiments, a stimulation range for the treatment session is set that starts with the threshold of phosphenes and is increased in the session or subsequent sessions, to a level up to nine (9) times the threshold level when measured in micro-amps. In some embodiments, the threshold level of electrical stimulation and the highest level of electrical stimulation are both within a range of about 100 to about 1000 microamps.

In some embodiments, the highest level of stimulation a patient can tolerate is linked to the highest level of stimulation that occurs when the patient continues to see phosphenes but does not have discomfort. In some embodiments, the electrical stimulation is ramped up until patient discomfort is reported (or objectively observed) and then backed off to as high a level as possible without such discomfort (in some embodiments, that stimulation level is used as the upper range for treatment in the session). In some embodiments, each subsequent treatment session of the electrical stimulation is recalibrated based upon the patient's sighting of phosphenes and levels of discomfort. In some embodiments, each eye is also individually calibrated within the treatment session since one eye may be able to tolerate more stimulation than the other. In other embodiments, both eyes are calibrated at the same settings in order to simplify the clinical procedures associated with the electrical stimulation.

In some embodiments, the present invention combines a regularly repeating monophasic waveform with an overlaid biphasic waveform within the same electrical stimulation treatment session. In some embodiments, the combination of monophasic and biphasic waveforms achieves both cellular re-energization and cellular repair, a duality which neither alone can achieve, and in some embodiments, both cellular re-energization and cellular repair are required to restore patient vision and to halt the progression of eye disease such as dry AMD. In some embodiments, a monophasic waveform is used simultaneously with a biphasic waveform in the same electrical stimulation treatment session. In some embodiments, a monophasic waveform is alternated with a biphasic waveform in the same electrical stimulation treatment session. In some such embodiments, the monophasic and biphasic waveforms are alternated within 15 minutes of each other. In some embodiments, the waveforms used in the present invention operate at a pulse-repetition rate of between about 20 pulses per second to about 100 pulses per second. In some embodiments, each waveform operates at a single pulse-repetition rate. As used herein, "pulse-repetition rate" is informally referred to herein as frequency and/or Hz.

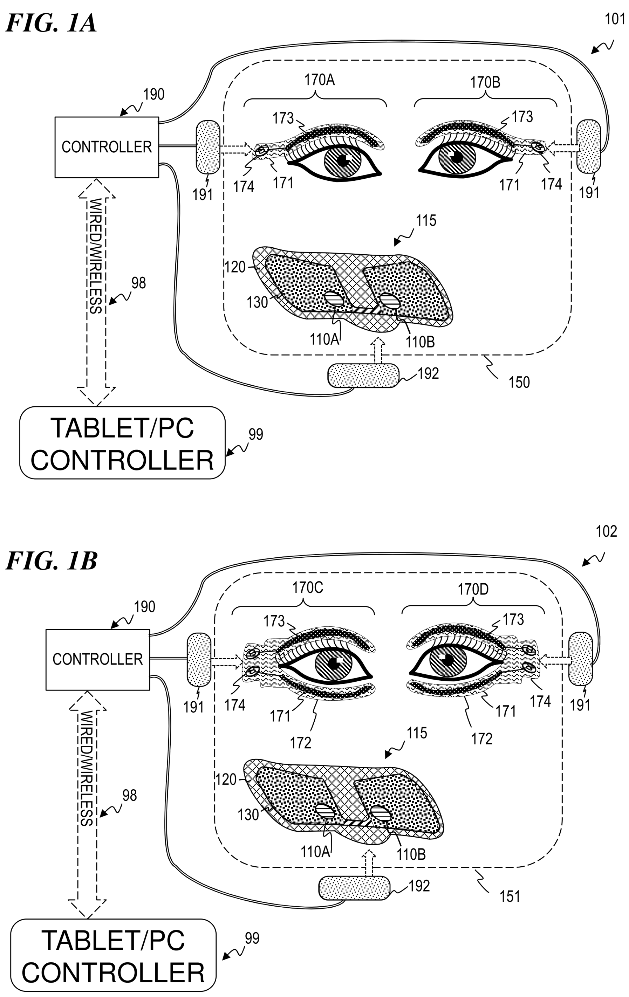

Figure 1A is a perspective-view diagram of an eye-stimulation system 101 that uses an eye-stimulation electrode system 150, according to some embodiments of the present invention. In some embodiments, eye-stimulation electrode system 150 includes return-electrode system 115 (such as described in PCT Patent Application publication WO 2021/231496, which is incorporated by reference above) and two eye-electrode systems 170A and 170B for the patient's right and left eyes, respectively. In some embodiments, return-electrode system 115 includes a single electrically conductive metallic electrode 130 having one or more openings through which one or more snap-type electrical connections 110A and 110B are assembled. In some embodiments, electrically conductive metallic electrode 130 is deposited on insulating polymer substrate 120. In some embodiments, return-electrode system 115 is placed on the back of the patient's head or neck to provide a pathway for stimulation signals to run through the eye from eye-electrode systems 170A and/or 170B to return-electrode system 115. In some embodiments, electrical connector 192 provides electrical connectivity between controller 190 and return-electrode system 115, while electrical connectors 191 each respectively provides electrical connectivity between controller 190 and eye-electrode systems 170A and 170B. In some embodiments, controller 190 supplies pulsed signals to connector 192 and the two connectors 191 that provide electrical stimulation through the upper eyelids of the patient to provide therapeutic stimulation designed to counteract eye diseases such as age-related macular degeneration (AMD). In some embodiments, the polarity of the signals is periodically reversed to reduce charge buildup in the tissues of the patient. In some embodiments, the magnitude of the signals when reversed is changed (e.g., longer duration or more numerous pulses at a lower magnitude of electrical current being used to neutralize charge buildup of shorter duration or less numerous pulses at a higher magnitude of electrical current). In some embodiments, a pulse envelope is applied to amplitude modulate a carrier of higher-frequency shorter pulses. In some embodiments, the pulse envelope is gradually ramped to higher magnitude intensity so as to avoid unpleasant sensory nerve stimulation. In some embodiments, each eye-electrode subsystem 170A, 170B includes an upper eyelid electrode 173 deposited as metal layers on a flexible insulating substrate 171, each electrode having a respective snap-type electrical connector 174 (or other suitable connector). In some embodiments, connectors 191 each have a corresponding receiver connector for each connector 174.

In some embodiments, using three separate parts, as is the case of eye-stimulation electrode system 150, allows easier application of the parts since they can be applied one at a time and accommodate easily to different head sizes. In some embodiments, each of the three parts has an individual QR-type code printed thereon (or, in other embodiments (not shown), a chip having such encoded serial number information), so that a camera application ("app") (or other suitable software app) on a smartphone or tablet 99 can read the respective QR codes to track which electrodes were used on which patient at what date and time for such purposes as tracking therapy (so adjustments can be made at future therapy sessions) and/or patient/insurance billing or the like. In some embodiments, tablet/PC controller 99 is coupled to controller 190 via a wired or wireless connection 98.

Figure 1B is a perspective-view diagram of an eye-stimulation system 102 that uses an eye-stimulation electrode system 151, according to some embodiments of the present invention. In some embodiments, eye-stimulation system 102 is operated in a manner substantially similar to the operation of eye-stimulation system 101 described above, except that eye-stimulation electrode system 151 includes eye-electrode systems 170C and 170D for the patient's right and left eyes, respectively. In some embodiments, each eye-electrode subsystem 170C, 170D includes a lower-eye-lid electrode 172 and an upper eyelid electrode 173 deposited as metal layers on a flexible insulating substrate 171, each electrode having a respective snap-type electrical connector 174 (or other suitable connector). In some embodiments, connectors 191 each have a corresponding receiver connector for each connector 174.

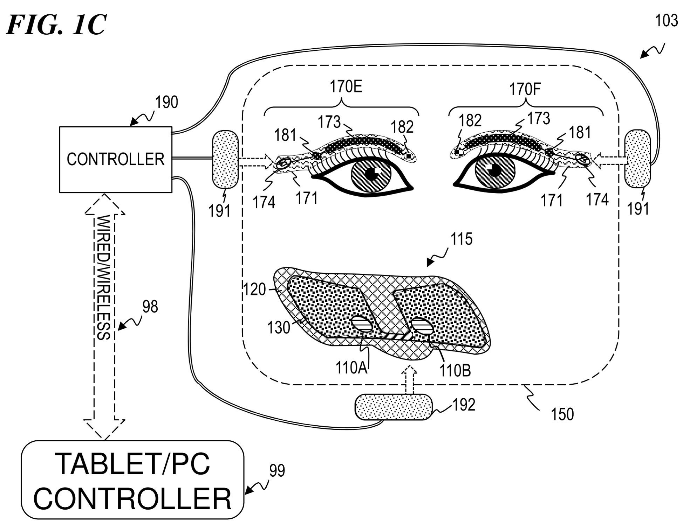

Figure 1C is a perspective-view diagram of an eye-stimulation system 103, according to some embodiments of the present invention. In some embodiments, system 103 is substantially similar to system 101 of Figure 1A except that system 103 includes eye-electrode systems 170E and 170F for the patient's right and left eyes, respectively, which each include one or more sensors 181 (e.g., electrode sensors that detect electrical signals of a nerve in the patient's eyelid skin) and one or more light emitters 182 (e.g., light-emitting diodes - LED(s)).

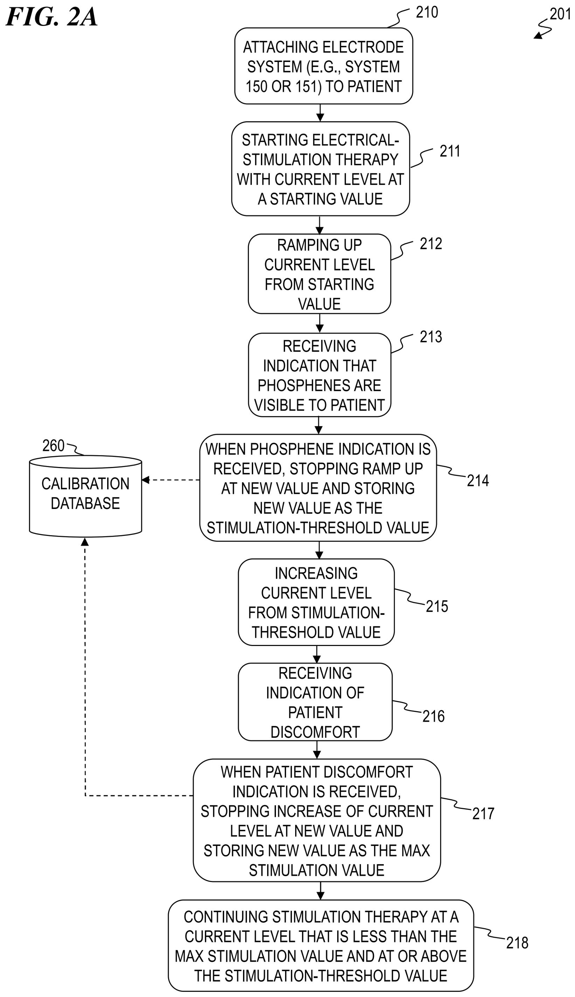

Figure 2A is a block diagram/flow chart of an eye-stimulation system and method 201 for electrical stimulation applied to human eyes, according to some embodiments of the present invention. In some embodiments, at block 210, an electrode system (e.g., in some embodiments, electrode system 150 of Figure 1A or electrode system 151 of Figure 1B that are part of overall stimulation systems 101 or 102, respectively) is attached to one or more eyes of the patient. In some embodiments, the electrical-stimulation therapy is started for at least one of the patient's eyes at block 211, and as part of the starting of the therapy, the current level is set at a starting value. In some embodiments, at block 212, the current level is ramped up from the starting value. In some embodiments, at block 213, an indication that phosphenes are visible to the patient in the eye being stimulated is received (e.g., in some embodiments, the phosphene indication is received directly from the patient; in some embodiments, the phosphene indication is received at controller 190 as a signal sent by sensors 181 of Figure 1C or the systems shown in Figures 6A-7; in still other embodiments, the presence of phosphenes is detected by sensor(s) 181 of Figure 1C, a signal is sent from sensor(s) 181 to light emitter(s) 182, and light is emitted from light emitter(s) 182 based on the signal from sensor(s) 181). In some embodiments, when the phosphene indication is received, the ramping up of the current level is stopped and the new value of the current level is stored to calibration database 260 as the stimulation-threshold value at block 214 (in some embodiments, database 260 is operatively coupled to controller 190 and/or tablet/PC 99 of Figure 1A). In some embodiments, at block 215, the current level is then increased from the stimulation-threshold value. In some embodiments, at block 216, an indication of patient discomfort is received (e.g., in some embodiments, the indication of patient discomfort comes directly from the patient; in some embodiments, the indication of patient discomfort is received at controller 190 as a signal sent by sensor(s) 181 of Figure 1C or the systems shown in Figures 6A-7; in still other embodiments, the presence of patient discomfort is detected by sensor(s) 181 of Figure 1C, a signal is sent from sensor(s) 181 to light emitter(s) 182, and light is emitted from light emitter(s) 182 based on the signal from sensor(s) 181). In some embodiments, at block 217, when the patient-discomfort indication is received, the increase of the current level is stopped and the new value of the current level is stored to calibration database 260 as the maximum (max) stimulation value. In some embodiments, the stimulation therapy is continued at block 218 at a current level that is less than the max stimulation value and at or above the stimulation-threshold value.

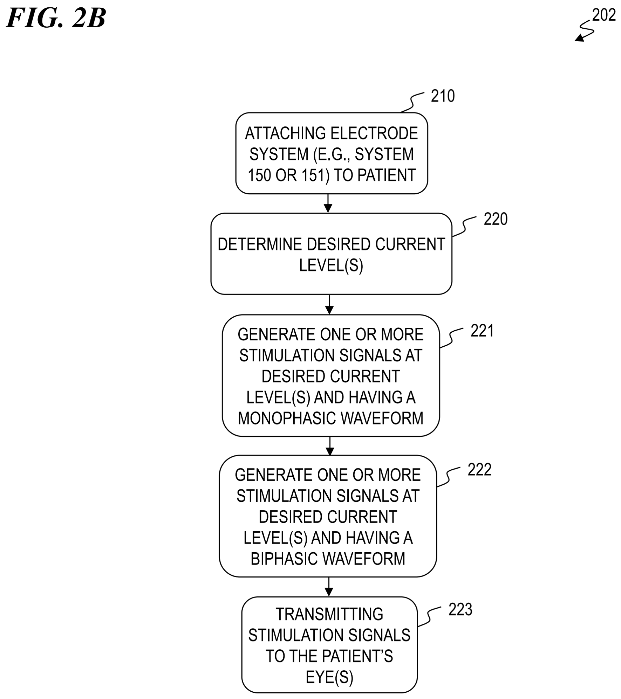

Figure 2B is a block diagram/flow chart of an eye-stimulation system and method 202 for electrical stimulation applied to human eyes, according to some embodiments of the present invention. In some embodiments, at block 220, the desired current level(s) for the electrical stimulation is determined (e.g., in some embodiments, the desired current level is selected to be at or above the stimulation-threshold level calculated in blocks 211-214 of Figure 2A and below the maximum stimulation level calculated in blocks 215-217 of Figure 2A). In some embodiments, at block 221, one or more stimulation signals are generated at the desired current level(s) and having a monophasic waveform. In some embodiments, at block 222, one or more stimulation signals are generated at the desired current level(s) and having a biphasic waveform. In some embodiments, at block 223, the stimulation signals are transmitted to the patient's eye(s). In some embodiments, the monophasic waveform signals are transmitted at a first time, the biphasic waveform signals are transmitted at a second time, and the first time doesn't overlap with the second time (e.g., in some embodiments, the monophasic waveform signals are separated in time from the biphasic waveform signals by no more than fifteen (15) minutes). In some embodiments, the monophasic waveform signals are transmitted at least partially simultaneously with the transmission of the biphasic waveform signals.

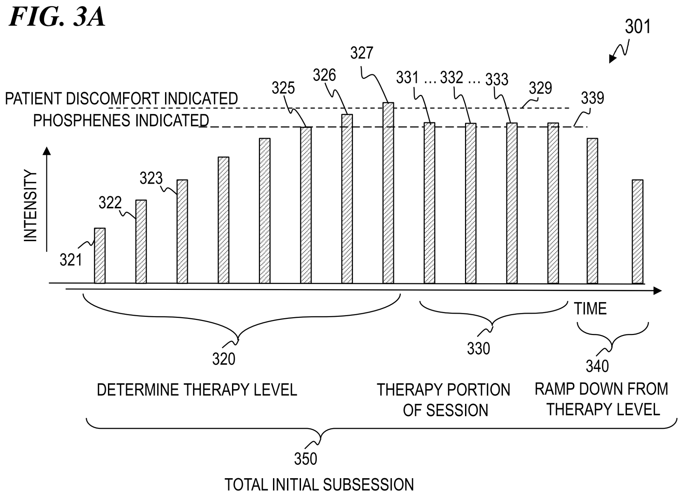

Figure 3A is a graph 301 of electrical signal amplitude over time as applied to an eye of a patient during an initial sub-session 350 used to determine an amplitude of signal that is both effective and not too uncomfortable to the patient, according to some embodiments of the present invention. In some embodiments, a stimulation-threshold series of pulses 320 are generated that have gradually increasing electrical current amplitude (see pulses 321, 322, and 323, etc., wherein the thin diagonal lines shown on each pulse (e.g., pulses 321, 322, 323, and 331, 332, 333, etc.) do not indicate separate micro pulses, but rather are merely shading of each single pulse) until controller 190 (see, e.g., Figure 1A) receives feedback as to the skin impedance or electrical signals from a nerve in the eyelid skin (or electrical signals from the cornea directly, from the visual cortex region of the brain, or the like) and/or feedback from an optical sensor monitoring the size of the pupil and/or patient-generated feedback that indicates that phosphenes are visible to the patient. In some embodiments, when this feedback is received, the current amplitude is set as the stimulation-threshold level. In some embodiments, the electrical current amplitude is then further increased until controller 190 receives feedback as to the skin impedance or electrical signals from a nerve in the eyelid skin and/or patient-generated feedback (such as a voice command (or involuntary vocalization) or a push-button switch signal) that indicates patient discomfort (see, e.g., pulse 327 that is above the discomfort level 329). In some embodiments, upon determining the discomfort level 329, controller 190 reduces the current and/or voltage of the pulses to a therapeutic level 339 that is better tolerated than the level causing discomfort. In some embodiments, a therapy series of pulses 330 is then applied, e.g., therapy pulses 331, 332, 333, etc. In some embodiments, a ramp-down series of pulses 340 is applied after the therapy series of pulses 330. In some embodiments, initial sub-session 350 includes the stimulation-threshold series of pulses 320, the therapy series of pulses 330, and the ramp-down series of pulses 340.

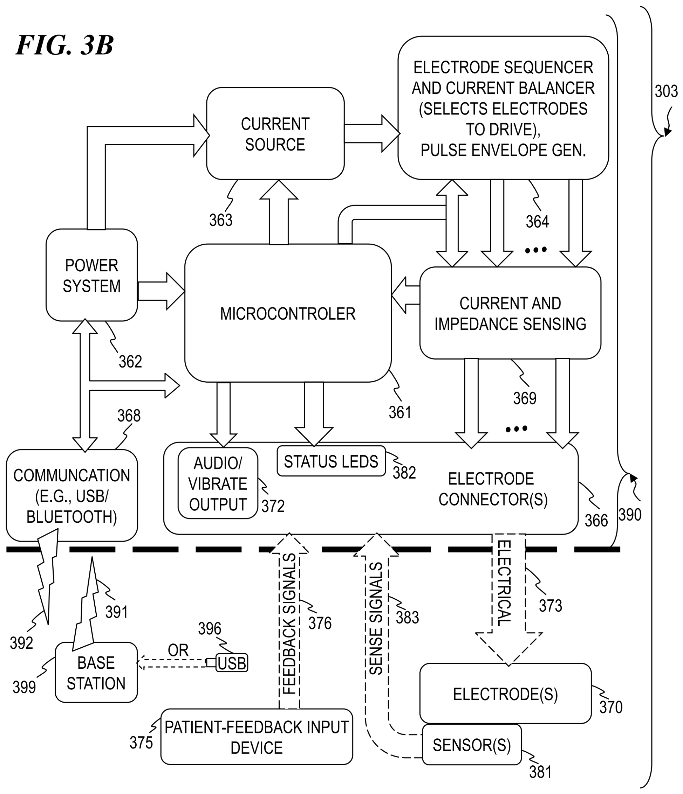

Figure 3B is a block diagram of a system 303 that can vary amplitude and/or waveform of signals over time as applied to an eye of a patient, such that the amplitude and/or waveform of the signals is both effective and not too uncomfortable to the patient according to some embodiments of the present invention. In some embodiments, system 303 includes base station 399, controller 390, FLASH drive 396, electrodes 370 (e.g., in some embodiments, electrodes 170A and 170B of Figure 1A), and (as needed) ground patches (e.g., in some embodiments, return-electrode system 115 of Figure 1A), conductive gel and cleaning wipes. In some embodiments, base station 399 is a device, such as a laptop personal computer (PC), tablet computer, desktop computer or the like (e.g., in some embodiments, tablet/PC controller 99 of Figure 1A), for selecting parameters, monitoring performance, data collection and storage and communication with the controller 390 (e.g., in some embodiments, controller 190 of Figure 1A). In some embodiments, controller 390 is a control unit that contains the electronics that deliver current to the electrode contacts on the eye. In some embodiments, the electrode contacts are part of a disposable strip, goggles or an individual probe or the like. In some embodiments, FLASH drive 396 is a USB "thumb drive" that includes encrypted data and program code to provide a fixed number of prepaid patient therapies, wherein each time a successful therapy is completed one therapy unit is deducted from the flash drive. In some embodiments, FLASH drive 396 is a USB "thumb drive" that includes encrypted data and program code to provide prescriptions for specific patient therapies, wherein each time a successful therapy is completed one therapy unit is deducted from the flash drive. In some embodiments, once all available therapy unit sessions are completed, the FLASH drive 396 can be discarded and a new prepaid FLASH drive is used. In other embodiments, the FLASH drive 396 is also used to gather and record session data and parameters that can be later analyzed to determine long-term effectiveness of various different therapy variations, so once all available therapy unit sessions are completed, the FLASH drive 396 is returned to the analysis facility and in exchange for the data and a per-therapy-session fee, a new prepaid flash drive is sent out to the treatment facility. In some embodiments, the patient-identification data is anonymized and encrypted for patient privacy and/or legal requirements, while keeping each session with enough information to analyze what works and what does not work. In some embodiments, electrodes 370 include a first electrode on a first adhesive strip that is placed on at least one of the upper eyelid and the lower eyelid of a first eye, and a second electrode on a second adhesive strip that is placed on at least one of the upper eyelid and the lower eyelid of a second eye. In some embodiments, a kit is provided wherein, in addition to the above-mentioned electrodes 370, one or more handheld probe tips, and/or goggles and the flash drive, the kit also includes such items as disposable ground patches, conductive gel and cleaning wipes.

In some embodiments, controller 390 includes a microprocessor 361, a power system (such as a battery, ultra-capacitor or the like) 362 that supplies electrical power to the rest of the controller 390, a current-source 363 that is controlled by microprocessor 361 based on signals from current and impedance sensor 369, an electrode sequencer and current balancer 364 that selects which electrodes to send the electrical pulse signal at any moment in time, as controlled by microprocessor 361, and these pulses are sent through electrode connectors 366 to electrodes 370. In some embodiments, sequencer and current balancer 364 also controls/generates a pulse envelope associated with the electrical stimulation treatment. In some embodiments, electrodes 370 also includes one or more status LEDs 382 embedded in or on the strip, wherein status LEDs 382 are driven by electrical signals sent through connector 366. In other embodiments, one or more status LEDs 382 are located in the controller 390 and emit light directly from controller 390, and/or through optical fibers 371 or the like embedded in or on the strip to emission points on the electrode strip, wherein these LEDs 382 are driven by electrical signals from microprocessor 361. In some embodiments, status LEDs 382 provide a status and patient-feedback function to tell the medical-professional person and/or the patient that the system is functioning and active. In some embodiments, system 303 includes one or more sensors 381 located on the patient that detect neural activity from the patient during stimulation, and send sense signals 383 based on the detected neural activity to controller 390. In some embodiments, status LEDs 382 are operatively coupled to sensors 381 and provide an indication that phosphenes are visible to the patient during stimulation and/or that the patient is experiencing discomfort based on the sense signals 383 received by controller 390. In some embodiments, system 303 includes a patient-feedback input device 375 (e.g., a push button, switch, or other suitable user-interface input device) operated by the patient to provide patient-generated feedback signals 376 to controller 390. In some embodiments, patient-generated feedback signals 376 indicate that phosphenes are visible to the patient and/or that the patient is experiencing discomfort (see, e.g., pulse 327 that is above the discomfort level 329 in Figure 3A). In some embodiments, status LEDs 382 are operatively coupled to patient-feedback input device 375 and are configured to emit light based on the received patient-generated feedback signals 376.

In some embodiments, a wireless communications device 368 (such as Bluetooth^{®}, NFC, infrared optical communications, or the like) provides one-way or two-way communications to a base station 399. In some embodiments, base station 399, based on a prepaid therapy authorization from, e.g., FLASH drive 396, transmits 391 programming information specific for the particular patient, wherein the authorization optionally includes authorization based on a fee having been paid, as well as patient-specific therapy control information that has been customized for the particular identified patient to be treated this session based on a treatment regimen prescribed by an eye doctor or the like. In some embodiments, session parameters are communicated 392 back to the base station 399 (with parameters such as the actual number, polarity, sequence and strength of pulses, the measured impedance and/or current, indicated patient discomfort, and the like). In some embodiments, system 303 includes a patient-activatable switch (e.g., on controller 390 or via a separate handheld switch that is wirelessly or in wired communication with controller 390) that the patient is instructed to press if and when the patient feels discomfort or concern, and upon activation of that switch, electrical output from controller 390 or even the entire controller 390 is immediately shut off, and/or the timing of the activation of the switch by the patient is recorded and transmitted in the communication 392 of parameters from the session. Thus, this feedback from the patient herself or himself, in some embodiments, is used to fully shut down the device (for patient comfort and peace-of-mind, as well as a further enhancement to patient safety just in case the current source 363 has a fault and is sending too much current), and is then correlated to a particular time or other aspect of the treatment to allow design of better therapy sessions in the future, and/or can be used to immediately terminate the session (wherein microcontroller 361 will immediately change all connections to "OFF" (or high impedance) to block any further current to the patient, and/or the entire controller 390 is then (i.e., after storing the timestamp of the switch press by the patient) shut down and disconnected from power source (e.g., battery) 362. In some embodiments, controller 390 and/or base station 399 include an audio/vibration-output unit 372 that provides a sound (beep, chime, ding, or the like) and/or vibration associated with therapy session status, to indicate, e.g., "ON/session starting," "in therapy," "an alert as to insufficient or inappropriate treatment," and "OFF/session ending."

In some embodiments, system 303 is a software-driven system that provides programmability of all parameters including frequency, waveform, current level, duration of therapy and number of "cycles" around the eye (wherein, in some embodiments, when the adhesive strip on a given eye includes a plurality of electrodes 370, one cycle is the independent activation of each of the individual electrodes that make up the plurality of electrodes 370). In some embodiments, these parameters are programmed during manufacturing, while in other embodiments, the parameters are programmed in the field by the clinician or a company representative. In some embodiments, modifications to the programming parameters and/or software (e.g., as customized by the prescription for the treatment protocol provided by a licensed medical professional for a specific identified patient) are stored in a plug-in storage device 396 (such as a USB FLASH storage device or the like) and the parameters and/or program and loaded (by plugging-in device 396) into base station 399 (and then transmitted 391 (e.g., wirelessly or by wired connection) to controller 390 to be stored in the memory of microprocessor 361). In other embodiments, plug-in storage device 396 is plugged directly into controller 390 to load and store the parameters and/or program into the memory of microprocessor 361 (in some such embodiments, the base station 399 is omitted, while in other embodiments, base station 399 is retained to provide the technician/medical professional with status of each session in real time). In some embodiments, base station 399 is used to provide the technician/medical professional with status of each session of a plurality of simultaneous patient sessions in real time (e.g., in some embodiments, a laptop computer used as base station 399 is programmed to provide a split-screen progress monitor (e.g., wherein the display screen is split into, e.g., quadrants if up to four patients were simultaneously treated) for a plurality of treatment sessions for each of a plurality of patients). In some embodiments, the software may also be modified remotely using the wireless connection to the base station 399. In some embodiments, a prescription for a treatment session (the protocol, parameters and the like for controlling current amount, pulse duration, inter-pulse spacing and how many pulses are to be sent and the like) for each individual patient is prepared and checked by a licensed professional, and this prescription is downloaded and/or stored in base station 399, or into USB device 396 along with the prepaid activation code to enable only authorized treatments for specific patients. In some embodiments, the software in base station 399 and/or the software in controller 390 verifies the match between a specific patient's prescription associated with a specific identified patient and patient-identification information of the specific identified patient in order to verify that the correct prescription is used for that patient.

Some embodiments include a large memory in the controller 390 and/or in the base station 399 to capture and record all pertinent patient and clinic data, including the treatment protocol such as the number of pulses applied to each electrode, the amount of current, and all other relevant parameters of what the treatment session involved (including, for example, whether an actual or sham treatment session was provided to the particular patient). In some embodiments, the recorded data are stored in a permanent-memory portion of USB storage device 396 (e.g., using a portion of memory that allows only a single write operation that may be followed by many read operations, in order that the data are permanently stored and later available). In some embodiments, these data are collected remotely and summarized by company and/or clinic personnel. In some embodiments, data is summarized to provide comparisons between patients and clinics and may be used in research. Over time, this data will allow the company or analysis facility to optimize the design and the clinical protocol, thus improving outcomes.

Some embodiments provide greater current-drive capacity via current source 363, as well as better current and impedance measurements via sensor unit 369. This allows the controller 390 to deliver greater, and more-carefully controlled, current levels that overcome any unexpected higher impedance levels. In some embodiments, system 303 has a governor (e.g., current controller) to prevent delivery of more than 350 microamps (µA) to the patient during therapy. In some embodiments, base station 399 and/or controller 390 may be activated only via an appropriately encoded message from flash drive 396, or via an authentic encrypted code (e.g., in some embodiments, received from a company website on the internet) that enables the laptop to signal, via WI-FI in some embodiments, the microstimulation controller 361 to conduct the therapy session for a particular identified patient. In some embodiments, controller 390 may be activated via a flash drive 396 plugged into controller 390 or by any other suitable type of connection (such as a USB cable to base station 399).

Some embodiments provide automatic adjustment to changes in impedance. As impedance changes during treatment, from contact to contact and from eye to eye, the control unit 390 will automatically adjust to maintain a consistent current level. This improves performance and outcomes. The treatment has been automated to minimize clinician involvement. The system 303 automatically manages the therapy to ensure uniform and repeatable results.

In some embodiments, the control unit 390 is designed to fit and connect nicely on the ground patch(es). This eliminates the potential of losing signal to the left and right set of contacts due to patient movement during therapy. The small size of the control unit reduces clutter, improves patient comfort, and improves device consistency and compliance. In some embodiments, the control unit 390 is designed to be tamper proof (both physically and electronically), and to provide encryption on the programming and the sensed parameters to prevent hacking.

In some embodiments, the base station 399 communicates with the control unit 390 via a wireless connection eliminating the need to tether the patient to the base station. This improves compliance and makes the setup and therapy session easier to manage. In some embodiments, the base station 399 can communicate with multiple control units at one time reducing the number of base stations required, therefore reducing set-up time and the clinician's time to manage multiple patients.

In some embodiments, multiple levels of protection help ensure that the electrical current delivered to the contacts cannot exceed the programmed current. The design ensures that an unsafe level of current cannot be achieved even if the output was shorted (zero impedance). In some embodiments, the control unit 390 is powered by a small direct-current (DC) button cell and is not connected to the base station during therapy, reducing or eliminating the possibility of injury to the patient. In some embodiments, the low cost of the design allows most or all of the system to be single-use and disposable. In some embodiments, the base station 399 can communicate with a device such as a goggle device and or strips partially or completely encircling the upper and or lower eyelids, as well as other body parts.

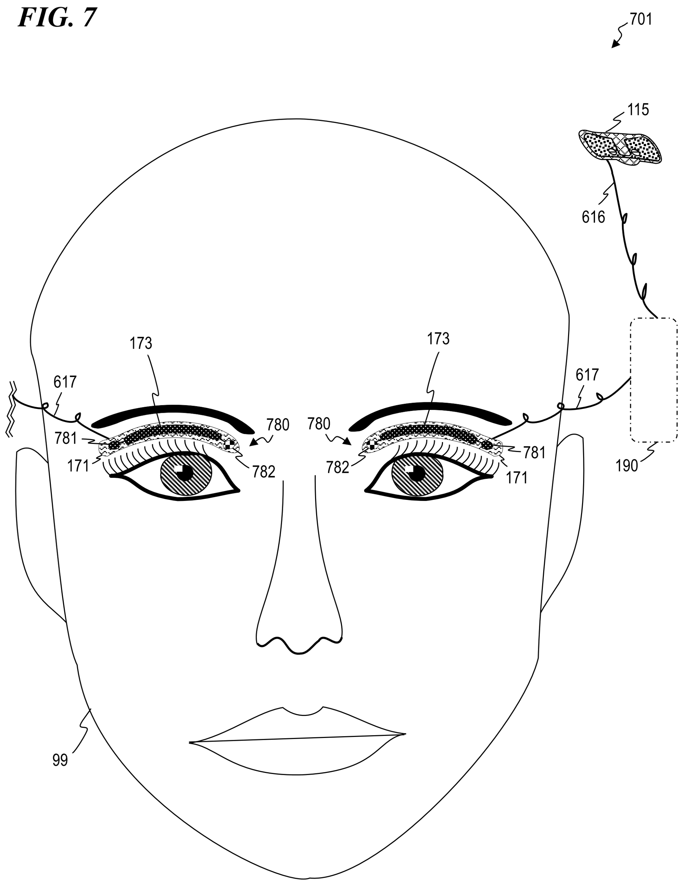

Figures 6A-7 show various configurations used to monitor the physiological response of the patient during electrical stimulation of the eye (e.g., in some embodiments, the configurations of Figures 6A-6D are used to observe electrical activity of neural cells in the retina, optic nerve, and/or the brain in response to electrical stimulation, while the configuration of Figure 7 is used to observe pupil responses to the electrical stimulation). In some embodiments, one or more of these configurations is used to objectively determine characteristics of the electrical stimulation such as the level of intensity necessary to achieve therapeutic electrical stimulation (e.g., in some such embodiments, one or more of the configurations in Figures 6A-7 provide objective feedback during stimulation that replaces or complements the subjective approach of eliciting and receiving feedback directly from the patient). In some embodiments, the upper eyelids of patient 99 are closed during stimulation/response monitoring. In other embodiments, the eyes are open during stimulation/response monitoring.

In some embodiments, the present invention acquires and processes neural activity signals to generate surrogate markers that are strongly correlated with phosphene production. For example, in some embodiments, the threshold electrical stimulation intensity for phosphene production is frequency dependent, and in some such embodiments, the lowest threshold occurs at or at about 20 hertz (Hz) and increases steadily as the frequency increases or decreases from 20 Hz. The effect of stimulation frequency on the evoked potential (i.e., the neural activity generated in response to stimulation) provides useful information to correlate with phosphene production. In some embodiments, a frequency sweep from 1 Hz to 40 Hz is performed with the evoked potential recorded at each frequency. In some embodiments, particular frequencies within the frequency sweep are selected to best sample the frequency response around 20 Hz. For example, in some embodiments, the frequency sweep includes frequencies of 1 Hz, 10 Hz, 15 Hz, 17 Hz, 19 Hz, 20 Hz, 21 Hz, 23 Hz, 25 Hz, 30 Hz, and 40 Hz. In some such embodiments, if a high degree of correlation between evoked potential and stimulation frequency is obtained at a given stimulation intensity, it is assumed that the phosphene threshold has been achieved at that stimulation intensity.

Phosphenes also depend on other stimulation pulse characteristics, such as whether the pulse is biphasic or monophasic and whether the active electrode at the cornea or eyelid is anodic or cathodic. In some embodiments, for a biphasic pulse, where, by definition, the electrode polarity switches between anodic and cathodic, phosphene occurrence depends on whether the leading phase of the biphasic pulse is anodic or cathodic. In either case, in some embodiments, it is important to record the stimulation pulse characteristics and to correlate such records with the occurrence of phosphenes. The higher the correlation between phosphene production and electrically evoked responses (i.e., the neural activity generated in response to stimulation) across factors such as frequency, amplitude, and polarity, the higher the confidence that electrically evoked responses are reliable indicators of success in targeting retinal structures. For example, in some embodiments, phosphenes occur at a given frequency and phase duration at an intensity level of 200 µA (microamps) with biphasic pulses having a leading cathodic phase, but phosphene occurrence requires an intensity level of 250 µA when the leading phase is anodic. In some embodiments, if the corresponding electrically evoked potential follows a similar differential sensitivity between the polarity of the leading phase, then the reliability of the electrically evoked potential as a surrogate for phosphene production is enhanced.

Even though the stimulation current provided by the present invention is in the microamp range, in some embodiments, the stimulation electrodes are small and have a high resistance and therefore the voltage required to drive the stimulation pulse through the skin can reach 10 to 20 volts (V). In contrast, in some embodiments, the evoked potential amounts to a few microvolts, a million times less than the stimulation pulse. The skin has capacitance and can therefore store charge for a relatively long time, and during this time, in some embodiments, the highly sensitive amplifier which is provided to recover the evoked potential can be overwhelmed. In some embodiments, the amplifier saturates and take some time to recover with the resulting amplifier output being an artifact which has no physiological meaning. In some embodiments, therefore, the present invention provides a means to suppress this artifact. In some such embodiments, a balanced biphasic stimulation pulse is used to ensure that the net current delivered is zero, which minimizes the charge that is stored in the skin capacitance. In other such embodiments, the input of the amplifier is short circuited during the stimulation pulse, and shortly afterwards, to prevent it from entering a saturation mode. In other such embodiments, a blanking period of, for example, 5 to 50 milliseconds (ms) is provided during which the recovered signal is ignored.

The subject/patient can communicate the perception of a phosphene verbally and indeed can describe its location and other characteristics such as flash, streak, color. In some embodiments, it is useful to correlate these subjective observations with sensor data. In some embodiments, a microphone is provided to capture voice responses from the subject, together with voice processing software which categories the response to record when the phosphene was reported and its characteristics. In some such embodiments, the system correlates these reports with the stimulation characteristics such as frequency, pulse type, amplitude, pulse phase charge. In this way the relationship between the occurrence of specific phosphene types can be correlated with specific stimulation characteristics for an individual on a particular occasion. In some embodiments, data from the same subject recovered on different occasions is compared to track changes and in some embodiments, data from a population of subjects is pooled for statistical analysis.

In some embodiments, a hand switch is provided which allows the subject to report the occurrence of a phosphene. In some such embodiments, the stimulation system includes an input to capture the moment the phosphene was reported and therefore correlate with stimulation parameters as described above. In some embodiments, multiple keys or buttons are provided to expand on the options to record a phosphene (in some embodiments, for example, a pattern of five keys allows the user to report phosphene position in the field of vision as up, down, left, right, and center).

In some embodiments, the measurement of electrical potentials at the body surface arising from electrical activity originating within the body requires at least two electrodes and the electrical potential is the voltage difference, usually time varying, between the at least two electrodes. In some embodiments, a third electrode is used to combat common-mode interference. In some embodiments of the two-sensor system, the two sensor electrodes are located in a position that best captures particular neural responses of the vision system. In some embodiments, the interpretation of the electrical signals received by the sensor electrodes depends, at least in part, on the specific location of the sensor electrodes.

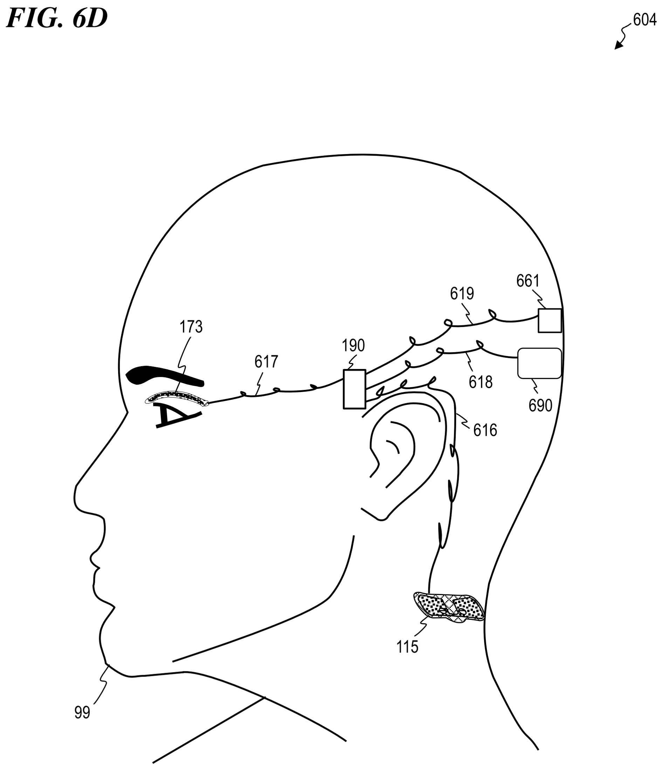

Visually Evoked Responses (VER) include electrical activity collected in response to flashes or images presented to the retina and therefore provide insight into photoreceptor and signal transmission in the vision system. In some embodiments, electrical stimulation of the retina such as provided by the present invention results in detectable electrical signals at the visual cortex that resemble VER even where the photoreceptors are not functioning, which implies that the electrical stimulation is capable of activating neural cells further downstream in the vision pathway. Accordingly, in some embodiments, electrical signals generated at the visual cortex in response to electrical stimulation provide another form of neural activity or electrically evoked responses (EER) monitored by the present invention. In some embodiments, for example, the present invention provides sensor-electrode pairs placed on the scalp over the visual cortex to capture electrical activity in the brain resulting from signals coming in from the optic nerve (see, e.g., Figure 6D). This captured electrical activity provides valuable information on the functioning of the visual pathway.

In some embodiments, the present invention provides a sensor-electrode pair that includes one electrode at the cornea (or on the eyelid) and a second electrode located at, for example, the side of the head on the temple in order to capture electrical potentials originating within the orbit of the eye (see, e.g., Figures 6A-6C). In some such embodiments, the sensor-electrode pair provides electroretinogram (ERG) data.

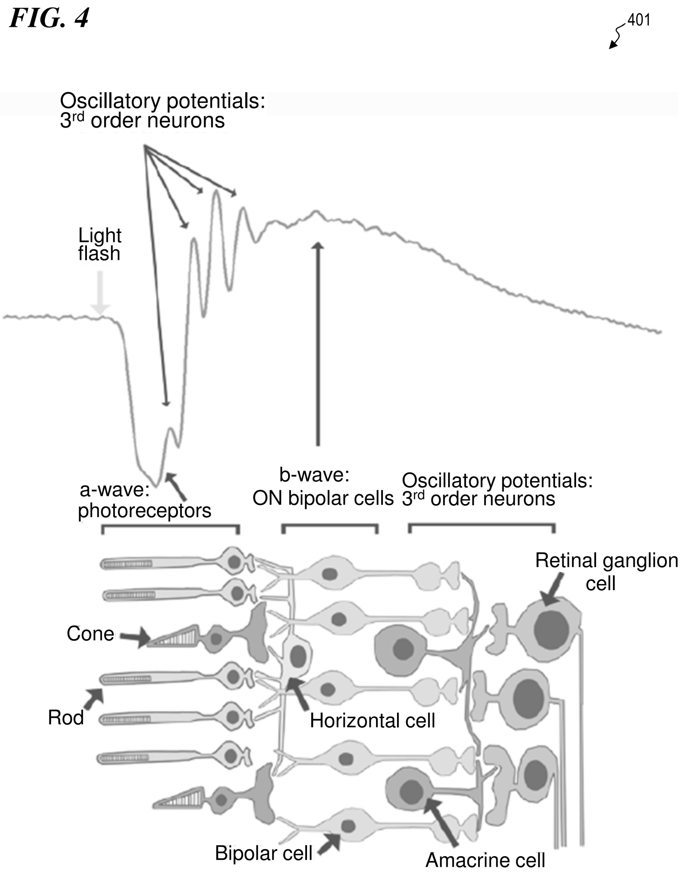

Figure 4 is an example electroretinogram (ERG) 401 generated in response to a light flash. ERG 401 also provides the location in the retinal cells of the individual ERG components. The x-axis of ERG 401 represents time in milliseconds (e.g., time latency from the light flash) and the y-axis represents amplitude of the ERG in microvolts (µV). As shown in Figure 4, the a-wave component of the ERG is generated by the rod and cone photoreceptors on the outer layer of the retina while the b-wave component of the ERG originates in the bipolar cells of the retina.

In some embodiments, electrically evoked responses captured by the present invention include a number of peaks, or components, of either polarity, and in some embodiments, these components have characteristic time latencies, measured in the millisecond range, with respect to the stimulus event that elicited them. In some such embodiments, normal ranges for such latencies have been established and measurements outside these ranges help with the diagnosis of certain conditions. In some embodiments, the individual components of electrically invoked responses are associated with specific neural structures and so reduced or delayed peaks point to deficiencies in these structures. In some embodiments, individual components are numbered P1, P2, etc. for positive, and N1, N2, etc. for negative. In some embodiments, the individual components are referred to by the letters a, b, c, or by their expected latency (e.g., P75 for a positive component with 75 milliseconds of latency). In some embodiments, the individual components of the electrically evoked responses provide indicators to classify the electrically evoked responses.

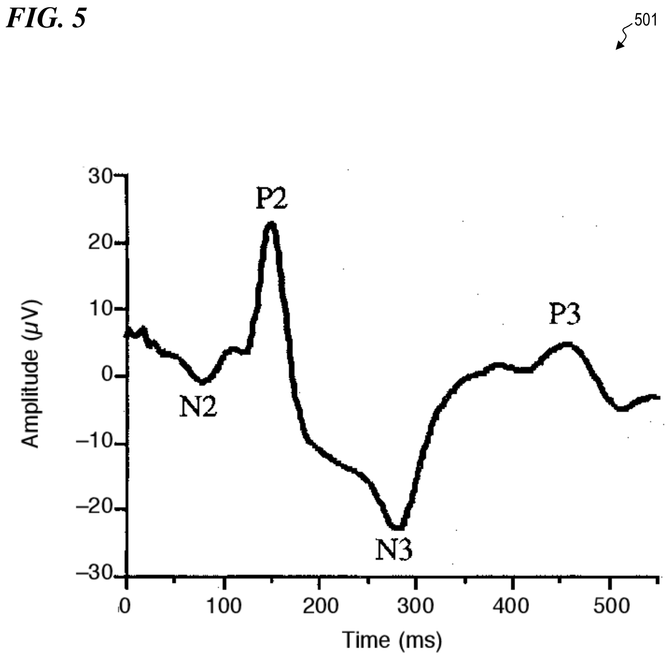

Figure 5 is a graph 501 showing individual components of a visually evoked response (VER). As shown in graph 501, the x-axis represents time in milliseconds and the y-axis represents amplitude of the VER in microvolts (µV). In some embodiments, the latencies and relative amplitude of the components N2, P2, N3, and P3 in graph 501 are correlated with parallel components in an electrically evoked response caused by the electrical stimulation of the present invention.

In some embodiments, the present invention captures an electrically evoked response and classifies the recovered waveform by measuring the amplitude and latency of selected components of the waveform. In some embodiments, the electrical stimulation pulse applied to the eye is increased in intensity until a specific component of the response reaches a specific amplitude or latency threshold. In some such embodiments, the achievement of this threshold is interpreted as a successful stimulation of the target tissue in the retina. In some embodiments, signal processing techniques to recover and classify waveforms are provided and typically involve examining the waveform to isolate the characteristic components and then measuring their amplitude and time delay with respect to a datum. For example, in some embodiments, this is done by cross correlating the recovered time domain waveforms with template waveforms, or the use of matched filters. In some embodiments, frequency domain approaches are used to measure changes in the amplitude and phase of characteristic frequencies. In some embodiments, other pattern recognition and machine learning techniques are used.

Figure 6A is a schematic front-view diagram of an electrical stimulation system 601 positioned on patient 99, according to some embodiments of the present invention. In some embodiments of stimulation system 601, each eye includes an upper eyelid electrode 173 deposited as metal layers on a flexible insulating substrate 171. In some embodiments, stimulation system 601 further includes one or more return (ground)-electrode systems 115 placed on the back of the neck or head of patient 99 (or on any other suitable ground location on the patient), and one or more stimulation controllers 190 operatively coupled to both return-electrode system 115 and eyelid electrodes 173 via electrical connections 616 and 617, respectively (e.g., in some embodiments, one or more wires; in some embodiments, printed tracks on a substrate that is part of or connects to substrate 171). In some embodiments, the components of system 601 on each eye of patient 99 are controlled by a single therapy controller 190 and are coupled to a single return-electrode system 115. In other embodiments, each eye's components include a respective therapy controller 190 coupled to a corresponding return-electrode system 115. In some embodiments, controller 190 is located locally (e.g., in a battery-operated unit that is carried by patient 99, such as in a shirt pocket or head-mounted elastic band or in/on an cable-holder like, for example, cable-holder system 1501 of Figures 15A-15B of U.S. Patent 11,116,973, which is incorporated by reference above), while in other embodiments, controller 190 is attached to or part of a computer-controlled apparatus such as a laptop personal computer, a tablet computer, a desktop computer, a smartphone, or the like.

In some embodiments, system 601 includes a first sensor electrode 660 and a second sensor (reference) electrode 661 that are operatively coupled to controller 190 via electrical connections 618 and 619, respectively. In some embodiments, electrical-signal data is detected via first sensor electrode 660 and reference electrode 661 during electrical stimulation provided by eyelid electrodes 173, and the detected electrical-signal data is transmitted to controller 190 where it is processed and/or further transmitted to a remote computer device. In some embodiments, first sensor electrode 660 includes a gold foil electroretinogram (ERG) electrode (e.g., in some embodiments, a gold foil electrode such as described in the background of U.S. Patent 5,154,174, which is incorporated by reference above) that is (at least partially) inserted behind the lower eyelid such that first sensor electrode 660 contacts the conjunctiva (in some such embodiments, the exposed portion of first sensor electrode 660 is adhered to the skin below the eye). In some embodiments, first sensor electrode 660 includes a contact lens having an electrode on an outer surface to contact the subject's inner eyelid or conjunctiva. In some embodiments, first sensor electrode 660 includes any other suitable ERG electrode configured to contact the conjunctiva such as the electrode described and shown in the figures of U.S. Patent 5,154,174, which is incorporated by reference above. As shown in Figure 6A, reference electrode 661 is shown on the side of the head of patient 99, but, in other embodiments, reference electrode 661 is located in any location on patient 99 suitable for combining with first sensor electrode 660 to detect the desired electrical-signal data.

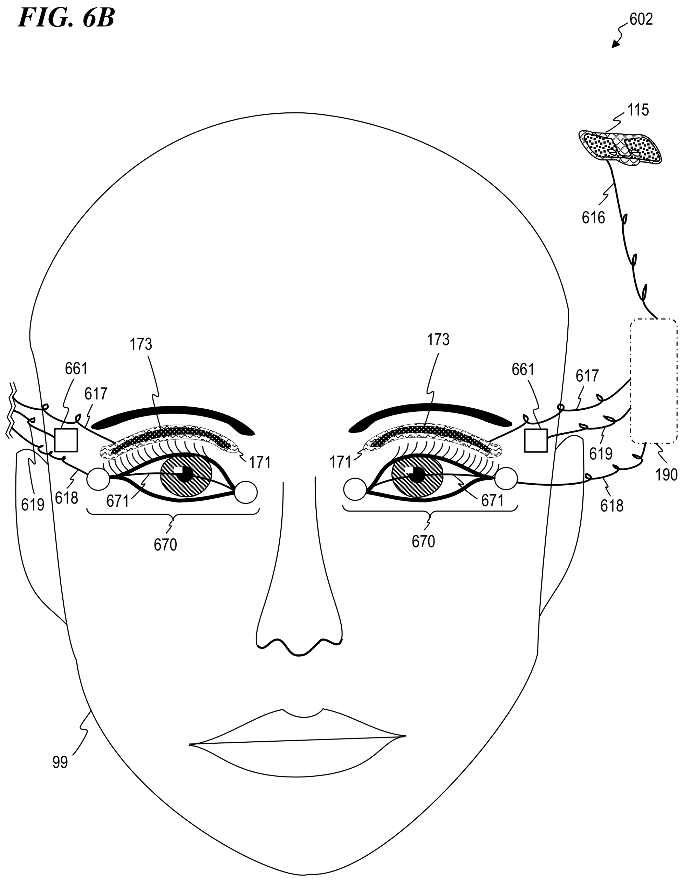

Figure 6B is a schematic front-view diagram of an electrical stimulation system 602 positioned on patient 99, according to some embodiments of the present invention. In some embodiments, system 602 is substantially similar to system 601 except that first sensor electrode 660 is replaced with a first sensor electrode 670. In some embodiments, first sensor electrode 670 includes a Dawson-Trick-Litzkow (DTL) ERG electrode that contacts the cornea with a fine wire 671 (e.g., a fine silver wire). In some embodiments, first sensor electrode 670 includes a DTL fine wire electrode or DTL fibre electrode such as described in the background of U.S. Patent 5,154,174, which is incorporated by reference above. In some embodiments, during stimulation from eyelid electrode 173, the upper eyelid closes over wire 671 of first sensor electrode 670. In some embodiments, as shown in Figure 6B, wire 671 of first sensor electrode 670 is placed across the middle of the cornea, and, in some embodiments, wire 671 remains in that position when the upper eyelid closes over wire 671 during stimulation from eyelid electrode 173. In other embodiments (not shown), wire 671 rests on the lower portion of the cornea with the upper eyelid closed over wire 671 during stimulation from eyelid electrode 173, and in still other embodiments (also not shown), wire 671 slides underneath the lower eyelid when the upper eyelid closes over wire 671 during stimulation from eyelid electrode 173.

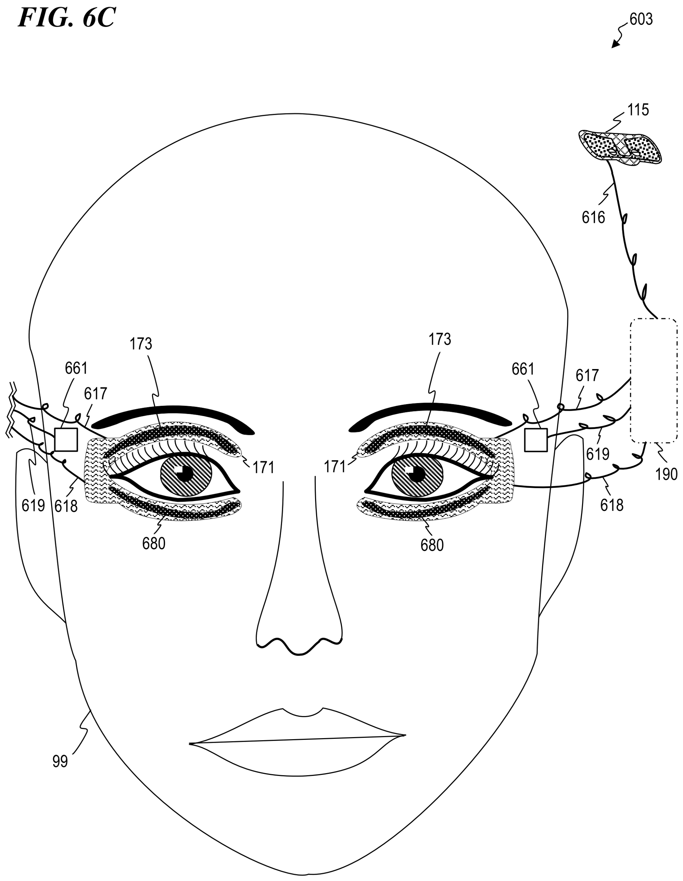

Figure 6C is a schematic front-view diagram of an electrical stimulation system 603 positioned on patient 99, according to some embodiments of the present invention. In some embodiments, system 603 is substantially similar to system 601 except that first sensor electrode 660 is replaced with a first sensor electrode 680 affixed to the lower eyelid of patient 99. In some embodiments, first sensor electrode 680 is on the same flexible insulating substrate 171 as upper eyelid electrode 173. In some embodiments, upper eyelid electrode 173 also functions as a sensing electrode by monitoring the retinal response to stimulation after eyelid electrode 173 delivers the stimulation pulse (and, in some embodiments, after any stimulation artifacts are suppressed).

Figure 6D is a schematic side-view diagram of an electrical stimulation system 604 positioned on patient 99, according to some embodiments of the present invention. In some embodiments, system 604 is substantially similar to system 601 except that first sensor electrode 660 is replaced with a first sensor electrode 690 that is placed at the back of the head of patient 99 near the visual cortex of the brain of patient 99 (in some such embodiments, first sensor electrode 690 is placed on the occipital scalp of patient 99). In some embodiments, reference electrode 661 is also moved to a position at the back of the head of patient 99 near the visual cortex of the brain of patient 99, while in other embodiments, reference electrode 661 is placed in any other suitable location on patient 99 including the temple, earlobe, mastoid, and the like. In some embodiments, first sensor electrode 690 and reference electrode 661 detect electrical activity generated in the brain of patient 99 (e.g., in the visual cortex of the brain) in response to stimulation provided by eyelid electrode 173.

Figure 7 is a schematic front-view diagram of an electrical stimulation system 701 positioned on patient 99, according to some embodiments of the present invention. In some embodiments, system 701 includes a pupil-measurement device 780 configured to help determine if the patient sees phosphenes during electrical stimulation. In some embodiments, the pupil response to the electrical stimulation provided by the present invention is correlated with phosphene production. In some such embodiments, the pupil constricts with the occurrence of phosphenes as though the phosphenes were real light (in some embodiments, the threshold intensity of electrical stimulation to elicit a pupil response (i.e., pupil constriction) is slightly higher than for the intensity to elicit actual phosphenes, but nonetheless, the pupil response provides a reliable phosphene indicator). Due to the pupil response being a consensual reflex (e.g., light directed into one eye produces pupil constriction in both eyes), the non-stimulated eye will also constrict if phosphenes are present in the stimulated eye, and therefore, in some embodiments, the pupil area of the opposite (non-stimulated) eye that remains open during stimulation of the stimulated eye is measured to provide an indication of phosphene visibility during electrical stimulation.

In some embodiments, the stimulated eye is closed during stimulation and direct measurement of the pupil of the stimulated eye is desired, and in some such embodiments, infrared measurement is used to estimate pupil area through the closed eyelid. The retina is a relatively strong source of infrared light and since the iris absorbs some of the emitted light, the aperture of the pupil can be assessed by measuring infrared light emitted by the eye (see, e.g., the paper by *Farraj et al.,* incorporated by reference above, which describes a means by which the orbit is illuminated through the temple with an infrared LED and the light emanating from the pupil is captured with an infrared camera). In some embodiments, the present invention provides an enhanced infrared-measurement technique by placing an infrared source at the eyelid and measuring the extent of backscattered radiation.

In some embodiments, pupil-measurement device 780 includes an infrared sensor device 781 positioned at the eyelid to measure the infrared light emitted from the pupil. In some such embodiments, it is only necessary to measure the relative constriction of the pupil and an absolute measure of pupil area is not necessary. In some embodiments, a threshold for pupil constriction of about 3% or greater is used as an indication of phosphenes being visible to the patient.

In some embodiments, pupil-measurement device 780 includes an infrared light-emitting diode (IR LED) 782 and an infrared (IR) sensor 781 integrated together with the stimulation electrode 173 on insulating substrate 171. In some embodiments, the IR LED 782 is used to illuminate the orbit of the eye being electrically stimulated. In some embodiments, the amount of backscattered light is continuously monitored within controller 190. In some embodiments, a stable pupil is indicated by a steady signal from sensor 781, and when the pupil constricts due to phosphenes, the signal from sensor 781 is reduced. In some embodiments, if the pupil response is relatively slow, the signal is low-pass filtered to about 2 Hz to reduce noise and still capture the low-frequency pupil response. In some embodiments, the electrical stimulation is incremented in a stepwise fashion while the pupil response is monitored, and each step is maintained for at least one second to allow detection of the pupil response. In some such embodiments, the stimulation level at which the response occurred is taken as the phosphene threshold.

In some embodiments (not shown), an IR camera is positioned in front of the eye and the area of the pupil is assessed. In some embodiments, a sustained reduction in area of about 3% or greater is deemed to be an evoked constriction and is used as an indication that phosphenes are visible to the patient.

In some embodiments, the present invention provides a method that includes providing a first electrode substrate, wherein the first electrode substrate includes: one or more electrodes on the first electrode substrate that are configured to apply electrical-stimulation therapy to a first eye of a patient, the first eye having an upper eyelid and a lower eyelid, wherein the first electrode substrate is configured to locate the one or more electrodes on an outer surface of at least one of the upper eyelid and the lower eyelid of the patient's first eye, attaching the first electrode substrate to the patient's skin such that the one or more electrodes are on the outer surface of at least one of the upper eyelid and the lower eyelid of the patient's first eye; applying the electrical-stimulation therapy to the first eye during a first treatment session, wherein the applying of the therapy to the first eye during the first treatment session includes: setting a current level of the therapy applied to the first eye at a first value, wherein the first value of the current level is below a stimulation threshold of the first eye, increasing the current level from the first value, and receiving a first indication that phosphenes are visible to the patient in the first eye, wherein the increasing of the current level from the first value is stopped at a second value when the first indication is received.

In some embodiments of the method, the receiving of the first indication includes eliciting and receiving the first indication from the patient. In some embodiments, the receiving of the first indication includes: providing one or more sensors operatively coupled to the first electrode substrate; and detecting, using the one or more sensors, electrical signals of a nerve from the patient's eyelid skin that indicate visibility of the phosphenes to the patient in the first eye. In some embodiments, the receiving of the first indication includes: providing one or more sensors operatively coupled to the first electrode substrate; providing a first light emitter operatively coupled to the sensors; detecting, using the one or more sensors, electrical signals of a nerve from the patient's eyelid skin that indicate visibility of the phosphenes to the patient in the first eye; and emitting light from the first light emitter based at least in part on the detected electrical signals. In some embodiments, the second value of the current level is at or above the stimulation threshold of the first eye.

In some embodiments of the method, the applying of the electrical-stimulation therapy to the first eye during the first treatment session further includes: increasing the current level from the second value to a third value, wherein the third value is no more than a multiple of nine (9) times the second value. In some embodiments, the applying of the electrical-stimulation therapy to the first eye during the first treatment session further includes: increasing the current level from the second value to a third value, wherein the second value and the third value are both within a range of about 100 microamps to about 1,000 microamps. In some embodiments, the applying of the electrical-stimulation therapy to the first eye during the first treatment session further includes: increasing the current level from the second value, and receiving a second indication that the patient is experiencing discomfort, wherein the increasing of the current level from the second value is stopped at a third value when the second indication is received.

In some embodiments of the method, the applying of the electrical-stimulation therapy to the first eye during the first treatment session further includes: increasing the current level from the second value, receiving a second indication that the patient is experiencing discomfort, wherein the increasing of the current level from the second value is stopped at a third value when the second indication is received, and reducing the current level from the third value to a fourth value that is less than the third value and at or above the second value. In some embodiments, the applying of the electrical-stimulation therapy to the first eye during the first treatment session further includes: increasing the current level from the second value, and receiving a second indication that the patient is experiencing discomfort, wherein the increasing of the current level from the second value is stopped at a third value when the second indication is received, and wherein the receiving of the second indication includes: providing one or more sensors operatively coupled to the first electrode substrate; and sensing, using the one or more sensors, electrical signals of a nerve from the patient's eyelid skin that indicate patient discomfort.

In some embodiments, the method further includes applying the electrical-stimulation therapy to the first eye during a second treatment session, wherein the applying of the therapy to the first eye during the second treatment session includes: setting the current level of the therapy applied to the first eye at the second value, and increasing the current level from the second value to a third value, wherein the third value is no more than a multiple of nine (9) times the second value.

In some embodiments, the method further includes applying the electrical-stimulation therapy to the first eye during a second treatment session, wherein the applying of the therapy during the second session includes: setting the current level of the therapy applied to the first eye at a fourth value, wherein the fourth value of the current level is below a stimulation threshold of the first eye, increasing the current level from the fourth value, receiving a third indication that phosphenes are visible to the patient in the first eye, wherein the increasing of the current level from the fourth value is stopped at a fifth value when the third indication is received, increasing the current level from the fifth value, and receiving a fourth indication that the patient is experiencing discomfort, wherein the increasing of the current level from the fifth value is stopped at a sixth value when the fourth indication is received.

In some embodiments, the method further includes providing a second electrode substrate, wherein the second electrode substrate includes: one or more electrodes on the second electrode substrate that are configured to apply electrical-stimulation therapy to a second eye of the patient, the second eye having an upper eyelid and a lower eyelid, wherein the second electrode substrate is configured to locate the one or more electrodes on an outer surface of at least one of the upper eyelid and the lower eyelid of the patient's second eye, attaching the second electrode substrate to the patient's skin such that the one or more electrodes of the second electrode substrate are on the outer surface of at least one of the upper eyelid and the lower eyelid of the patient's second eye; applying the electrical-stimulation therapy to the second eye during the first treatment session, wherein the applying of the therapy to the second eye during the first treatment session includes: setting a current level of the therapy applied to the second eye at the second value, and increasing the current level from the second value to a third value, wherein the third value is no more than a multiple of nine (9) times the second value.

In some embodiments, the method further includes providing a second electrode substrate, wherein the second electrode substrate includes: one or more electrodes on the second electrode substrate that are configured to apply electrical-stimulation therapy to a second eye of the patient, the second eye having an upper eyelid and a lower eyelid, wherein the second electrode substrate is configured to locate the one or more electrodes on an outer surface of at least one of the upper eyelid and the lower eyelid of the patient's second eye, attaching the second electrode substrate to the patient's skin such that the one or more electrodes of the second electrode substrate are on the outer surface of at least one of the upper eyelid and the lower eyelid of the patient's second eye; applying the electrical-stimulation therapy to the second eye during the first treatment session, wherein the applying of the therapy to the second eye during the first treatment session includes: setting a current level of the therapy applied to the second eye at a seventh value, wherein the seventh value of the current level is below a stimulation threshold of the second eye, increasing the current level from the seventh value, receiving a fifth indication that phosphenes are visible to the patient in the second eye, wherein the increasing of the current level from the seventh value is stopped at an eighth value when the fifth indication is received.

In some embodiments, the method further includes providing a first return electrode; and attaching the first return electrode to a portion of the patient's neck, wherein the applying of the electrical-stimulation therapy to the first eye during the first treatment session further includes controlling an electrical current between at least one of the one or more electrodes on the first electrode substrate and the first return electrode such that the electrical current passes through a retina of the first eye of the patient. In some embodiments, the applying of the electrical-stimulation therapy to the first eye during the first treatment session includes: generating a first stimulation signal, wherein the first stimulation signal includes a monophasic waveform; transmitting the first stimulation signal to the first eye via the one or more electrodes on the first electrode substrate; generating a second stimulation signal, wherein the second stimulation signal includes a biphasic waveform; and transmitting the second stimulation signal to the first eye via the one or more electrodes on the first electrode substrate, wherein the transmitting of the first stimulation signal occurs at least partially simultaneously with the transmitting of the second stimulation signal. In some embodiments, the applying of the electrical-stimulation therapy to the first eye during the first treatment session includes: generating a first stimulation signal, wherein the first stimulation signal includes a monophasic waveform; transmitting the first stimulation signal to the first eye during a first time period via the one or more electrodes on the first electrode substrate; generating a second stimulation signal, wherein the second stimulation signal includes a biphasic waveform; and transmitting the second stimulation signal to the first eye during a second time period via the one or more electrodes on the first electrode substrate, wherein the second time period does not overlap with the first time period.

In some embodiments of the method, the applying of the electrical-stimulation therapy to the first eye during the first treatment session includes: generating a first stimulation signal, wherein the first stimulation signal includes a monophasic waveform; transmitting the first stimulation signal to the first eye during a first time period via the one or more electrodes on the first electrode substrate; generating a second stimulation signal, wherein the second stimulation signal includes a biphasic waveform; and transmitting the second stimulation signal to the first eye during a second time period via the one or more electrodes on the first electrode substrate, wherein the second time period does not overlap with the first time period, and wherein the first time period is separated from the second time period by no more than fifteen (15) minutes.

In some embodiments of the method, the applying of the electrical-stimulation therapy to the first eye during the first treatment session includes: generating a plurality of stimulation signals, wherein the plurality of stimulation signals operate in a frequency range of about 20 to about 100 Hz; and transmitting the plurality of stimulation signals to the first eye via the one or more electrodes on the first electrode substrate.

In some embodiments, the present invention provides a system that includes a first electrode substrate that includes: one or more electrodes on the first electrode substrate that are configured to apply electrical-stimulation therapy to a first eye of a patient, the first eye having an upper eyelid and a lower eyelid, wherein the first electrode substrate is configured to locate the one or more electrodes on an outer surface of at least one of the upper eyelid and the lower eyelid of the patient's first eye, a stimulation controller configured to control the electrical-stimulation therapy, wherein each of the one or more electrodes is operatively coupled to the stimulation controller, wherein the stimulation controller is further configured to: set a current level of the electrical-stimulation therapy at a first value during a first treatment session, wherein the first value of the current level is below a stimulation threshold of the first eye, increase the current level from the first value during the first treatment session, and receive a first indication during the first treatment session that phosphenes are visible to the patient in the first eye, wherein the increase of the current level from the first value is stopped at a second value when the first indication is received.

In some embodiments of the system, the stimulation controller is further configured to elicit and receive the first indication from the patient. In some embodiments, the system further includes one or more sensors operatively coupled to the stimulation controller, wherein the one or more sensors are configured to: detect electrical signals of a nerve from the patient's eyelid skin that indicate visibility of the phosphenes to the patient in the first eye, and transmit the detected electrical signals to the stimulation controller to form the first received indication. In some embodiments, the stimulation controller is further configured to: increase the current level from the second value during the first treatment session, and receive a second indication that the patient is experiencing discomfort during the first treatment session, wherein the increase of the current level from the second value is stopped at a third value when the second indication is received, the system further including sensors operatively coupled to the stimulation controller, wherein the one or more sensors are configured to: detect electrical signals of a nerve from the patient's eyelid skin that indicate patient discomfort, and transmit the detected electrical signals to the stimulation controller to form the second received indication.

In some embodiments of the system, the stimulation controller is further configured to: set the current level of the therapy applied to the first eye at the second value during a second treatment session, and increase the current level from the second value to a third value during the second treatment session, wherein the third value is no more than a multiple of nine (9) times the second value. In some embodiments, the system further includes a first return electrode configured to be located on a portion of the patient's neck, wherein the stimulation controller is further configured to control an electrical current between at least one of the one or more electrodes on the first electrode substrate and the first return electrode such that the electrical current passes through a retina of the first eye of the patient.

In some embodiments, the present invention provides a non-transitory computer-readable medium having instructions stored thereon for causing a suitably programmed control system to execute a method of providing an electrical-stimulation therapy to a first eye of a patient, the first eye having an upper eyelid and a lower eyelid, wherein the method is executed by the control system on an electrical-stimulation system that includes one or more electrodes, wherein the one or more electrodes are on a first electrode substrate, wherein the first electrode substrate is attached to the patient's skin such that the one or more electrodes are on the outer surface at least one of the upper eyelid and the lower eyelid of the patient's first eye, the method including: applying the electrical-stimulation therapy to the first eye during a first treatment session, wherein the applying of the therapy to the first eye during the first treatment session includes: setting a current level of the therapy at a first value, wherein the first value of the current level is below a stimulation threshold of the first eye, increasing the current level from the first value, and receiving a first indication that phosphenes are visible to the patient in the first eye, wherein the increasing of the current level from the first value is stopped at a second value when the first indication is received.

In some embodiments, the present invention provides a system for delivering electrical-stimulation therapy to a patient, the system including a first electrode substrate that includes: one or more stimulation electrodes on the first electrode substrate that are configured to apply electrical stimulation signals to a first eye of the patient, the first eye having an upper eyelid and a lower eyelid, wherein the first electrode substrate is configured to locate the one or more electrodes on an outer surface of at least one of the upper eyelid and the lower eyelid of the first eye of the patient; a plurality of sensor electrodes configured to detect electrical activity from neural cells of the patient during delivery of the electrical-stimulation therapy, wherein the plurality of sensor electrodes includes a first sensor electrode configured to generate active electrical-activity data and a second sensor electrode configured to generate reference electrical-activity data; a first return electrode configured to be placed in a location on the patient that provides a return path for the electrical stimulation signals applied by the one or more stimulation electrodes such that an electrical current is formed between at least one of the one or more stimulation electrodes and the first return electrode; and a stimulation controller operatively coupled to the one or more stimulation electrodes, the plurality of sensor electrodes, and the first return electrode, wherein the stimulation controller is configured to control the electrical current formed between the at least one of the one or more stimulation electrodes and the first return electrode such that the electrical current passes through a retina of the first eye of the patient and delivers the electrical-stimulation therapy.

In some embodiments of the system, the electrical activity detected by the plurality of sensor electrodes includes electroretinogram (ERG) data. In some embodiments, the first sensor electrode includes a gold foil electrode configured to contact a conjunctiva of the first eye of the patient. In some embodiments, the first sensor electrode includes a Dawson-Trick-Litzkow (DTL) electrode configured to contact a cornea of the first eye of the patient. In some embodiments, the first sensor electrode is configured to be placed on the outer surface of the lower eyelid of the first eye of the patient. In some embodiments, the second sensor electrode is located on a side of a head of the patient.

In some embodiments of the system, the stimulation controller is further configured to compare the active electrical-activity data generated by the first sensor electrode to the reference electrical-activity data generated by the second sensor electrode in order to determine a characteristic of the electrical-stimulation therapy (e.g., in some embodiments, the stimulation controller includes a differential amplifier that detects the difference in voltage between the first sensor electrode and the second sensor electrode). In some embodiments, the stimulation controller is further configured to: set a current level of the electrical-stimulation therapy at a first value during a first treatment session, wherein the first value of the current level is below a stimulation threshold of the first eye, increase the current level from the first value during the first treatment session, and compare, during the first treatment session, the active electrical-activity data generated by the first sensor electrode to the reference electrical-activity data generated by the second sensor electrode in order to determine a characteristic of the electrical-stimulation therapy during the first treatment session, wherein the characteristic includes a first indication that phosphenes are visible to the patient in the first eye during the first treatment session, and wherein the increase of the current level from the first value is stopped at a second value when the first indication is determined.

In some embodiments, the system further includes a user interface configured to elicit and receive feedback from the patient during the first treatment session, wherein the stimulation controller is calibrated for the determination of the first indication based at least in part on the feedback received by the user interface. In some embodiments, the stimulation controller is further configured to increase the current level from the second value during the first treatment session, wherein the characteristic of the electrical-stimulation therapy includes a second indication that the patient is experiencing discomfort during the first treatment session, and wherein the increase of the current level from the second value is stopped at a third value when the second indication is determined. In some embodiments, the stimulation controller is further configured to: set a current level of the electrical-stimulation therapy at a first value during a first treatment session, wherein the first value of the current level is below a stimulation threshold of the first eye, increase the current level from the first value during the first treatment session, and elicit and receive, from the patient, a first indication that phosphenes are visible to the patient, wherein the increase of the current level from the first value is stopped at a second value when the first indication is received.

In some embodiments, the system further includes a second electrode substrate that includes: one or more stimulation electrodes on the second electrode substrate that are configured to apply electrical stimulation signals to a second eye of the patient, the second eye having an upper eyelid and a lower eyelid, wherein the second electrode substrate is configured to locate the one or more electrodes on an outer surface of at least one of the upper eyelid and the lower eyelid of the second eye of the patient; wherein the first return electrode is further configured to provide a return path for the electrical stimulation signals applied by the one or more stimulation electrodes on the second electrode substrate such that an electrical current is formed between at least one of the one or more stimulation electrodes on the second electrode substrate and the first return electrode; wherein the stimulation controller is operatively coupled to the one or more stimulation electrodes on the second electrode substrate; and wherein the stimulation controller is further configured to control the electrical current formed between the at least one of the one or more stimulation electrodes on the second electrode substrate and the first return electrode such that the electrical current passes through a retina of the second eye of the patient and delivers the electrical-stimulation therapy.

In some embodiments, the present invention provides a method for delivering electrical-stimulation therapy to a patient, the method including providing a first electrode substrate, wherein the first electrode substrate includes: one or more stimulation electrodes on the first electrode substrate that are configured to apply electrical stimulation signals to a first eye of the patient, the first eye having an upper eyelid and a lower eyelid; attaching the first electrode substrate to the patient's skin such that the one or more stimulation electrodes are on the outer surface of at least one of the upper eyelid and the lower eyelid of the first eye of the patient; providing a plurality of sensor electrodes including a first sensor electrode and a second sensor electrode; providing a first return electrode; placing the first return electrode in a location on the patient that provides a return path for the electrical stimulation signals applied by the one or more stimulation electrodes such that an electrical current is formed between at least one of the one or more stimulation electrodes and the first return electrode; delivering the electrical-stimulation therapy to the first eye, wherein the delivering includes controlling the electrical current formed between the at least one of the one or more stimulation electrodes and the first return electrode such that the electrical current passes through a retina of the first eye of the patient; and detecting, using the plurality of sensor electrodes, electrical activity from neural cells of the patient during the delivering of the electrical-stimulation therapy to the first eye, wherein the detecting of the electrical activity includes: generating active electrical-activity data using the first sensor electrode, and generating reference electrical-activity data using the second sensor electrode.

In some embodiments of the method, the detecting of the electrical activity includes generating electroretinogram (ERG) data. In some embodiments, the method further includes comparing the active electrical-activity data from the first sensor electrode to the reference electrical-activity data from the second sensor electrode in order to determine a characteristic of the electrical-stimulation therapy. In some embodiments, the method further includes setting a current level of the electrical-stimulation therapy at a first value during a first treatment session, wherein the first value of the current level is below a stimulation threshold of the first eye; increasing the current level from the first value during the first treatment session; comparing, during the first treatment session, the active electrical-activity data from the first sensor electrode to the reference electrical-activity data from the second sensor electrode; determining a characteristic of the electrical-stimulation therapy during the first treatment session based at least in part on the comparing, wherein the characteristic includes a first indication that phosphenes are visible to the patient in the first eye during the first treatment session; and stopping the increasing of the current level from the first value at a second value based on the first indication.

In some embodiments, the method further includes eliciting and receiving feedback from the patient during the first treatment session, wherein the determining of the first indication is calibrated based at least in part on the received feedback. In some embodiments, the method further includes increasing the current level from the second value during the first treatment session, wherein the characteristic includes a second indication that the patient is experiencing discomfort during the first treatment session; and stopping the increasing of the current level from the second value at a third value based on the second indication. In some embodiments, the method further includes placing the first sensor electrode on the patient such that at least a portion of the first sensor electrode contacts a conjunctiva of the first eye of the patient. In some embodiments, the method further includes placing the first sensor electrode on the patient such that at least a portion of the first sensor electrode contacts a cornea of the first eye of the patient. In some embodiments, the method further includes placing the first sensor electrode on the outer surface of the lower eyelid of the first eye of the patient.

In some embodiments, the present invention provides a non-transitory computer-readable medium having instructions stored thereon for causing a suitably programmed control system to execute a method for delivering electrical-stimulation therapy to a patient, the first eye having an upper eyelid and a lower eyelid, wherein the method is executed by the control system on an electrical-stimulation system that includes one or more stimulation electrodes on a first electrode substrate, wherein the one or more stimulation electrodes are configured to apply electrical stimulation signals to the first eye of the patient, wherein the first electrode substrate is attached to the patient's skin such that the one or more stimulation electrodes are on the outer surface at least one of the upper eyelid and the lower eyelid of the first eye of the patient, a plurality of sensor electrodes including a first sensor electrode and a second sensor electrode, a first return electrode that is placed in a location on the patient that provides a return path for the electrical stimulation signals applied by the one or more stimulation electrodes such that an electrical current is formed between at least one of the one or more stimulation electrodes and the first return electrode, the method including delivering the electrical-stimulation therapy to the first eye, wherein the delivering includes controlling the electrical current formed between the at least one of the one or more stimulation electrodes and the first return electrode such that the electrical current passes through a retina of the first eye of the patient; and detecting, via the plurality of sensor electrodes, electrical activity from neural cells of the patient during the delivering of the electrical-stimulation therapy to the first eye, wherein the detecting of the electrical activity includes: generating active electrical-activity data using the first sensor electrode, and generating reference electrical-activity data using the second sensor electrode.

In some embodiments, the non-transitory computer-readable medium further includes instructions such that the method further includes: setting a current level of the electrical-stimulation therapy at a first value during a first treatment session, wherein the first value of the current level is below a stimulation threshold of the first eye; increasing the current level from the first value during the first treatment session; comparing, during the first treatment session, the active electrical-activity data from the first sensor electrode to the reference electrical-activity data from the second sensor electrode; determining a characteristic of the electrical-stimulation therapy during the first treatment session based at least in part on the comparing, wherein the characteristic includes a first indication that phosphenes are visible to the patient in the first eye during the first treatment session; and stopping the increasing of the current level from the first value at a second value based on the first indication.

In some embodiments, the non-transitory computer-readable medium further includes instructions such that the method further includes suppressing electrical artifacts associated with the detected electrical activity. In some embodiments, the detected electrical activity includes a detected waveform, the non-transitory computer-readable medium further including instructions such that the method further includes measuring an amplitude and a latency of a plurality of components of the detected waveform, wherein the controlling of the electrical current includes increasing an intensity of the electrical current until a selected component of the plurality of components of the detected waveform reaches a threshold level for at least one of the amplitude and the latency.

In some embodiments, the present invention provides a system for delivering electrical-stimulation therapy to a patient, the system including a first electrode substrate that includes: one or more stimulation electrodes on the first electrode substrate that are configured to apply electrical stimulation signals to a first eye of the patient, the first eye having an upper eyelid and a lower eyelid, wherein the first electrode substrate is configured to locate the one or more electrodes on an outer surface of at least one of the upper eyelid and the lower eyelid of the first eye of the patient; a pupil-measurement device operatively coupled to the controller and configured to measure an aperture of a pupil of the first eye during delivery of the electrical-stimulation therapy; a first return electrode configured to be placed in a location on the patient that provides a return path for the electrical stimulation signals applied by the one or more stimulation electrodes such that an electrical current is formed between at least one of the one or more stimulation electrodes and the first return electrode; and a stimulation controller operatively coupled to the one or more stimulation electrodes, the plurality of sensor electrodes, and the first return electrode, wherein the stimulation controller is configured to control the electrical current formed between the at least one of the one or more stimulation electrodes and the first return electrode such that the electrical current passes through a retina of the first eye of the patient and delivers the electrical-stimulation therapy.

In some embodiments of the system, the stimulation controller is further configured to: set a current level of the electrical-stimulation therapy at a first value during a first treatment session, wherein the first value of the current level is below a stimulation threshold of the first eye, increase the current level from the first value during the first treatment session, and determine a characteristic of the electrical-stimulation therapy during the first treatment session based at least in part on the measured aperture of the pupil of the first eye, wherein the characteristic includes a first indication that phosphenes are visible to the patient in the first eye during the first treatment session, and wherein the increase of the current level from the first value is stopped at a second value when the first indication is determined. In some embodiments, the pupil-measurement device includes an infrared measurement device configured to measure infrared light emitted by the first eye.

In some embodiments, the present invention provides a system for delivering electrical-stimulation therapy to a patient, the system including a first electrode substrate that includes: one or more stimulation electrodes on the first electrode substrate that are configured to apply electrical stimulation signals to a first eye of the patient, the first eye having an upper eyelid and a lower eyelid, wherein the first electrode substrate is configured to locate the one or more electrodes on an outer surface of at least one of the upper eyelid and the lower eyelid of the first eye of the patient; a first return electrode operatively coupled to the one or more stimulation electrodes; means for controlling electrical current formed between the at least one of the one or more stimulation electrodes and the first return electrode; and means for detecting a biological response of the patient to the electrical-stimulation therapy during the delivering of the electrical-stimulation therapy to the first eye.

It is to be understood that the above description is intended to be illustrative, and not restrictive. Although numerous characteristics and advantages of various embodiments as described herein have been set forth in the foregoing description, together with details of the structure and function of various embodiments, many other embodiments and changes to details will be apparent to those of skill in the art upon reviewing the above description. The scope of the invention should be, therefore, determined with reference to the appended claims, along with the full scope of equivalents to which such claims are entitled. In the appended claims, the terms "including" and "in which" are used as the plain-English equivalents of the respective terms "comprising" and "wherein," respectively. Moreover, the terms "first," "second," and "third," etc., are used merely as labels, and are not intended to impose numerical requirements on their objects.

The specification and drawings of this divisional application are based on those of the parent application 22896495.3 (the content of which is here incorporated by reference). The following numbered clauses are based on the original claims of the parent application as filed. The following numbered clauses set out arrangements of the specification.

### Clauses:

Clause 1. A system for delivering electrical-stimulation therapy to a patient, the system comprising:
   a first electrode substrate that includes:
      one or more stimulation electrodes on the first electrode substrate that are configured to apply electrical stimulation signals to a first eye of the patient, the first eye having an upper eyelid and a lower eyelid, wherein the first electrode substrate is configured to locate the one or more electrodes on an outer surface of at least one of the upper eyelid and the lower eyelid of the first eye of the patient;
   a plurality of sensor electrodes configured to detect electrical activity from neural cells of the patient during delivery of the electrical-stimulation therapy, wherein the plurality of sensor electrodes includes a first sensor electrode placed in a first selected location on the patient and a second sensor electrode placed in a second selected location on the patient;
   a first return electrode configured to be placed in a return location on the patient that provides a return path for the electrical stimulation signals applied by the one or more stimulation electrodes such that an electrical current is formed between at least one of the one or more stimulation electrodes and the first return electrode; and
   a stimulation controller operatively coupled to the one or more stimulation electrodes, the plurality of sensor electrodes, and the first return electrode, wherein the stimulation controller is configured to control the electrical current formed between the at least one of the one or more stimulation electrodes and the first return electrode such that the electrical current passes through a retina of the first eye of the patient and delivers the electrical-stimulation therapy, and wherein the stimulation controller includes a differential amplifier configured to detect a difference in voltage between the first sensor electrode and the second sensor electrode in order to determine a characteristic of the electrical-stimulation therapy.
Clause 2. The system of clause 1, wherein the one or more stimulation electrodes also functions as one of the plurality of sensor electrodes.
Clause 3. The system of clause 1, wherein the first sensor electrode includes a gold foil electrode configured to contact a conjunctiva of the first eye of the patient.
Clause 4. The system of clause 1, wherein the first sensor electrode includes a Dawson-Trick-Litzkow (DTL) electrode configured to contact a cornea of the first eye of the patient.
Clause 5. The system of clause 1, wherein the first selected location of the first sensor electrode is the outer surface of the lower eyelid of the first eye of the patient.
Clause 6. The system of clause 1, wherein the second selected location of the second sensor electrode is on a side of the patient's head.
Clause 7. The system of clause 1, wherein the plurality of sensors includes a third sensor electrode configured to reduce common-mode interference.
Clause 8. The system of clause 1, wherein the stimulation controller is further configured to:
   set a current level of the electrical-stimulation therapy at a first value during a first treatment session, wherein the first value of the current level is below a stimulation threshold of the first eye, and
   increase the current level from the first value during the first treatment session, wherein the characteristic of the electrical-stimulation therapy determined by the stimulation controller includes a first indication that phosphenes are visible to the patient in the first eye during the first treatment session, and wherein the increase of the current level from the first value is stopped at a second value when the first indication is determined.
Clause 9. The system of clause 8, further comprising:
   a user interface configured to elicit and receive feedback from the patient during the first treatment session, wherein the stimulation controller is calibrated for the determination of the first indication based at least in part on the feedback received by the user interface.
Clause 10. The system of clause 8, wherein the stimulation controller is further configured to increase the current level from the second value during the first treatment session, wherein the characteristic of the electrical-stimulation therapy includes a second indication that the patient is experiencing discomfort during the first treatment session, and wherein the increase of the current level from the second value is stopped at a third value when the second indication is determined.
Clause 11. The system of clause 1, wherein the stimulation controller is further configured to:
   set a current level of the electrical-stimulation therapy at a first value during a first treatment session, wherein the first value of the current level is below a stimulation threshold of the first eye,
   increase the current level from the first value during the first treatment session, and
   elicit and receive, from the patient, a first indication that phosphenes are visible to the patient, wherein the increase of the current level from the first value is stopped at a second value when the first indication is received.
Clause 12. The system of clause 1, further comprising:
   a second electrode substrate that includes:
      one or more stimulation electrodes on the second electrode substrate that are configured to apply electrical stimulation signals to a second eye of the patient, the second eye having an upper eyelid and a lower eyelid, wherein the second electrode substrate is configured to locate the one or more electrodes on an outer surface of at least one of the upper eyelid and the lower eyelid of the second eye of the patient;
   wherein the first return electrode is further configured to provide a return path for the electrical stimulation signals applied by the one or more stimulation electrodes on the second electrode substrate such that an electrical current is formed between at least one of the one or more stimulation electrodes on the second electrode substrate and the first return electrode;
   wherein the plurality of sensor electrodes includes a third sensor electrode placed in a third selected location on the patient and a fourth sensor electrode placed in a fourth selected location on the patient;
   wherein the stimulation controller is operatively coupled to the one or more stimulation electrodes on the second electrode substrate, and wherein the stimulation controller is operatively coupled to the third sensor electrode and the fourth sensor electrode; and
   wherein the stimulation controller is further configured to control the electrical current formed between the at least one of the one or more stimulation electrodes on the second electrode substrate and the first return electrode such that the electrical current passes through a retina of the second eye of the patient and delivers the electrical-stimulation therapy, and wherein the differential amplifier is further configured to detect a difference in voltage between the third sensor electrode and the fourth sensor electrode in order to determine the characteristic of the electrical-stimulation therapy.
Clause 13. A method for delivering electrical-stimulation therapy to a patient, the method comprising:
   providing a first electrode substrate, wherein the first electrode substrate includes:
      one or more stimulation electrodes on the first electrode substrate that are configured to apply electrical stimulation signals to a first eye of the patient, the first eye having an upper eyelid and a lower eyelid;
   attaching the first electrode substrate to the patient's skin such that the one or more stimulation electrodes are on the outer surface of at least one of the upper eyelid and the lower eyelid of the first eye of the patient;
   providing a plurality of sensor electrodes including a first sensor electrode and a second sensor electrode;
   placing the first sensor electrode in a first selected location on the patient, and placing the second sensor electrode in a second selected location on the patient;
   providing a first return electrode;
   placing the first return electrode in a return location on the patient that provides a return path for the electrical stimulation signals applied by the one or more stimulation electrodes such that an electrical current is formed between at least one of the one or more stimulation electrodes and the first return electrode;
   delivering the electrical-stimulation therapy to the first eye, wherein the delivering includes controlling the electrical current formed between the at least one of the one or more stimulation electrodes and the first return electrode such that the electrical current passes through a retina of the first eye of the patient; and
   detecting, using the plurality of sensor electrodes, electrical activity from neural cells of the patient during the delivering of the electrical-stimulation therapy to the first eye, wherein the detecting of the electrical activity includes:
      detecting a difference in voltage between the first sensor electrode and the second sensor electrode in order to determine a characteristic of the electrical-stimulation therapy.
Clause 14. The method of clause 13, further comprising:
   generating electroretinogram (ERG) data based at least in part on the detected electrical activity.
Clause 15. The method of clause 13, wherein the plurality of sensor electrodes includes a third sensor electrode configured to reduce common-mode interference.
Clause 16. The method of clause 13, wherein the characteristic of the electrical-stimulation therapy includes a first indication that phosphenes are visible to the patient in the first eye during the first treatment session, the method further comprising:
   setting a current level of the electrical-stimulation therapy at a first value during a first treatment session, wherein the first value of the current level is below a stimulation threshold of the first eye;
   increasing the current level from the first value during the first treatment session; and
   stopping the increasing of the current level from the first value at a second value based on the first indication.
Clause 17. The method of clause 16, further comprising:
   eliciting and receiving feedback from the patient during the first treatment session, wherein the determining of the first indication is calibrated based at least in part on the received feedback.
Clause 18. The method of clause 16, further comprising:
   increasing the current level from the second value during the first treatment session, wherein the characteristic includes a second indication that the patient is experiencing discomfort during the first treatment session; and
   stopping the increasing of the current level from the second value at a third value based on the second indication.
Clause 19. The method of clause 13, wherein the placing of the first sensor electrode on the patient in the first selected location includes contacting a conjunctiva of the first eye of the patient with at least a portion of the first sensor electrode.
Clause 20. The method of clause 13, wherein the placing of the first sensor electrode on the patient in the first selected location includes contacting a cornea of the first eye of the patient with at least a portion of the first sensor electrode.
Clause 21. A non-transitory computer-readable medium having instructions stored thereon for causing a suitably programmed control system to execute a method for delivering electrical-stimulation therapy to a patient, the first eye having an upper eyelid and a lower eyelid, wherein the method is executed by the control system on an electrical-stimulation system that includes one or more stimulation electrodes on a first electrode substrate, wherein the one or more stimulation electrodes are configured to apply electrical stimulation signals to the first eye of the patient, wherein the first electrode substrate is attached to the patient's skin such that the one or more stimulation electrodes are on the outer surface at least one of the upper eyelid and the lower eyelid of the first eye of the patient, a plurality of sensor electrodes including a first sensor electrode placed in a first selected location on the patient and a second sensor electrode placed in a second selected location on the patient, a first return electrode that is placed in a return location on the patient that provides a return path for the electrical stimulation signals applied by the one or more stimulation electrodes such that an electrical current is formed between at least one of the one or more stimulation electrodes and the first return electrode, the method comprising:
   delivering the electrical-stimulation therapy to the first eye, wherein the delivering includes controlling the electrical current formed between the at least one of the one or more stimulation electrodes and the first return electrode such that the electrical current passes through a retina of the first eye of the patient; and
   detecting, via the plurality of sensor electrodes, electrical activity from neural cells of the patient during the delivering of the electrical-stimulation therapy to the first eye, wherein the detecting of the electrical activity includes:
      detecting a difference in voltage between the first sensor electrode and the second sensor electrode in order to determine a characteristic of the electrical-stimulation therapy.
Clause 22. The non-transitory computer-readable medium of clause 21, wherein the characteristic of the electrical-stimulation therapy includes a first indication that phosphenes are visible to the patient in the first eye during the first treatment session, wherein the non-transitory computer-readable medium further comprises instructions such that the method further includes:
   setting a current level of the electrical-stimulation therapy at a first value during a first treatment session, wherein the first value of the current level is below a stimulation threshold of the first eye;
   increasing the current level from the first value during the first treatment session; and
   stopping the increasing of the current level from the first value at a second value based on the first indication.
Clause 23. The non-transitory computer-readable medium of clause 21, further comprising instructions such that the method further comprises:
   suppressing electrical artifacts associated with the detected electrical activity.
Clause 24. The non-transitory computer-readable medium of clause 21, wherein the detected electrical activity includes a detected waveform, the non-transitory computer-readable medium further comprising instructions such that the method further comprises:
   measuring an amplitude and a latency of a plurality of components of the detected waveform, wherein the controlling of the electrical current includes increasing an intensity of the electrical current until a selected component of the plurality of components of the detected waveform reaches a threshold level for at least one of the amplitude and the latency.
Clause 25. A system for delivering electrical-stimulation therapy to a patient, the system comprising:
   a first electrode substrate that includes:
      one or more stimulation electrodes on the first electrode substrate that are configured to apply electrical stimulation signals to a first eye of the patient, the first eye having an upper eyelid and a lower eyelid, wherein the first electrode substrate is configured to locate the one or more electrodes on an outer surface of at least one of the upper eyelid and the lower eyelid of the first eye of the patient;
   a pupil-measurement device operatively coupled to the controller and configured to measure an aperture of a pupil of the first eye during delivery of the electrical-stimulation therapy;
   a first return electrode configured to be placed in a return location on the patient that provides a return path for the electrical stimulation signals applied by the one or more stimulation electrodes such that an electrical current is formed between at least one of the one or more stimulation electrodes and the first return electrode; and
   a stimulation controller operatively coupled to the one or more stimulation electrodes, the plurality of sensor electrodes, and the first return electrode, wherein the stimulation controller is configured to control the electrical current formed between the at least one of the one or more stimulation electrodes and the first return electrode such that the electrical current passes through a retina of the first eye of the patient and delivers the electrical-stimulation therapy.
Clause 26. The system of clause 25, wherein the stimulation controller is further configured to:
   set a current level of the electrical-stimulation therapy at a first value during a first treatment session, wherein the first value of the current level is below a stimulation threshold of the first eye,
   increase the current level from the first value during the first treatment session, and
   determine a characteristic of the electrical-stimulation therapy during the first treatment session based at least in part on the measured aperture of the pupil of the first eye, wherein the characteristic includes a first indication that phosphenes are visible to the patient in the first eye during the first treatment session, and wherein the increase of the current level from the first value is stopped at a second value when the first indication is determined.
Clause 27. The system of clause 25, wherein the pupil-measurement device includes an infrared measurement device configured to measure infrared light emitted by the first eye.

## Claims

1. A system for delivering electrical-stimulation therapy to a patient, the system comprising:
a first electrode substrate that includes:
one or more stimulation electrodes on the first electrode substrate that are configured to apply electrical stimulation signals to a first eye of the patient, the first eye having an upper eyelid and a lower eyelid, wherein the first electrode substrate is configured to locate the one or more electrodes at a front of the first eye of the patient, either directly on a cornea of the first eye or around the first eye of the patient;
a plurality of sensor electrodes configured to detect electrical activity from retinal or neural cells of the patient during delivery of the electrical-stimulation therapy, wherein the plurality of sensor electrodes includes a first sensor electrode placed in a first selected location on the patient and a second sensor electrode placed in a second selected location on the patient;
a first return electrode configured to be placed in a return location on the patient that provides a return path for the electrical stimulation signals applied by the one or more stimulation electrodes such that an electrical current is formed between at least one of the one or more stimulation electrodes and the first return electrode; and
a stimulation controller operatively coupled to the one or more stimulation electrodes, the plurality of sensor electrodes, and the first return electrode, wherein the stimulation controller is configured to control the electrical current formed between the at least one of the one or more stimulation electrodes and the first return electrode such that the electrical current passes through a retina of the first eye of the patient and delivers the electrical-stimulation therapy, and wherein the stimulation controller includes a differential amplifier configured to detect a difference in voltage between the first sensor electrode and the second sensor electrode in order to determine a characteristic of the electrical-stimulation therapy.

2. The system of claim 1, wherein the one or more stimulation electrodes also functions as one of the plurality of sensor electrodes.

3. The system of claim 1, wherein the first sensor electrode includes:
a) a gold foil electrode configured to contact a conjunctiva of the first eye of the patient; or
b) a Dawson-Trick-Litzkow (DTL) electrode configured to contact a cornea of the first eye of the patient.

4. The system of claim 1, wherein:
a) the first selected location of the first sensor electrode is the outer surface of the lower eyelid of the first eye of the patient; or
b) the second selected location of the second sensor electrode is on a side of the patient's head; or
c) the plurality of sensors includes a third sensor electrode configured to reduce common-mode interference.

5. The system of claim 1, wherein the stimulation controller is further configured to:
set a current level of the electrical-stimulation therapy at a first value during a first treatment session, wherein the first value of the current level is below a stimulation threshold of the first eye, and
increase the current level from the first value during the first treatment session, wherein the characteristic of the electrical-stimulation therapy determined by the stimulation controller includes a first indication that phosphenes are visible to the patient in the first eye during the first treatment session, and wherein the increase of the current level from the first value is stopped at a second value when the first indication is determined.

6. The system of claim 5, further comprising:
a user interface configured to elicit and receive feedback from the patient during the first treatment session, wherein the stimulation controller is calibrated for the determination of the first indication based at least in part on the feedback received by the user interface.

7. The system of claim 5, wherein the stimulation controller is further configured to increase the current level from the second value during the first treatment session, wherein the characteristic of the electrical-stimulation therapy includes a second indication that the patient is experiencing discomfort during the first treatment session, and wherein the increase of the current level from the second value is stopped at a third value when the second indication is determined.

8. The system of claim 1, wherein
a) the stimulation controller is further configured to:
set a current level of the electrical-stimulation therapy at a first value during a first treatment session, wherein the first value of the current level is below a stimulation threshold of the first eye,
increase the current level from the first value during the first treatment session, and
elicit and receive, from the patient, a first indication that phosphenes are visible to the patient, wherein the increase of the current level from the first value is stopped at a second value when the first indication is received; or
b) the first electrode substrate is configured to locate the one or more electrodes on an outer surface of at least one of the upper eyelid and the lower eyelid of the first eye of the patient.

9. The system of claim 1, further comprising:
a second electrode substrate that includes:
one or more stimulation electrodes on the second electrode substrate that are configured to apply electrical stimulation signals to a second eye of the patient, the second eye having an upper eyelid and a lower eyelid, wherein the second electrode substrate is configured to locate the one or more electrodes at a front of the second eye of the patient, either directly on a cornea of the second eye or around the second eye of the patient;
wherein the first return electrode is further configured to provide a return path for the electrical stimulation signals applied by the one or more stimulation electrodes on the second electrode substrate such that an electrical current is formed between at least one of the one or more stimulation electrodes on the second electrode substrate and the first return electrode;
wherein the plurality of sensor electrodes includes a third sensor electrode placed in a third selected location on the patient and a fourth sensor electrode placed in a fourth selected location on the patient;
wherein the stimulation controller is operatively coupled to the one or more stimulation electrodes on the second electrode substrate, and wherein the stimulation controller is operatively coupled to the third sensor electrode and the fourth sensor electrode; and
wherein the stimulation controller is further configured to control the electrical current formed between the at least one of the one or more stimulation electrodes on the second electrode substrate and the first return electrode such that the electrical current passes through a retina of the second eye of the patient and delivers the electrical-stimulation therapy, and wherein the differential amplifier is further configured to detect a difference in voltage between the third sensor electrode and the fourth sensor electrode in order to determine the characteristic of the electrical-stimulation therapy.

10. The system of claim 9, wherein the second electrode substrate is configured to locate the one or more electrodes on an outer surface of at least one of the upper eyelid and the lower eyelid of the second eye of the patient.

11. A non-transitory computer-readable medium having instructions stored thereon for causing a suitably programmed control system to execute a method for delivering electrical-stimulation therapy to a patient, a first eye having an upper eyelid and a lower eyelid, wherein the method is executed by the control system on an electrical-stimulation system that includes one or more stimulation electrodes on a first electrode substrate, wherein the one or more stimulation electrodes are configured to apply electrical stimulation signals to the first eye of the patient, wherein the first electrode substrate is attached to the patient's skin such that the one or more stimulation electrodes are at a front of the first eye of the patient, either directly on a cornea of the first eye or around the first eye of the patient, a plurality of sensor electrodes including a first sensor electrode placed in a first selected location on the patient and a second sensor electrode placed in a second selected location on the patient, a first return electrode that is placed in a return location on the patient that provides a return path for the electrical stimulation signals applied by the one or more stimulation electrodes such that an electrical current is formed between at least one of the one or more stimulation electrodes and the first return electrode, the method comprising:
delivering the electrical-stimulation therapy to the first eye, wherein the delivering includes controlling the electrical current formed between the at least one of the one or more stimulation electrodes and the first return electrode such that the electrical current passes through a retina of the first eye of the patient; and
detecting, via the plurality of sensor electrodes, electrical activity from retinal or neural cells of the patient during the delivering of the electrical-stimulation therapy to the first eye, wherein the detecting of the electrical activity includes:
detecting a difference in voltage between the first sensor electrode and the second sensor electrode in order to determine a characteristic of the electrical-stimulation therapy.

12. The non-transitory computer-readable medium of claim 11, wherein the first electrode substrate is configured to locate the one or more electrodes on an outer surface of at least one of the upper eyelid and the lower eyelid of the first eye of the patient.

13. A system for delivering electrical-stimulation therapy to a patient, the system comprising:
a first electrode substrate that includes:
one or more stimulation electrodes on the first electrode substrate that are configured to apply electrical stimulation signals to a first eye of the patient, the first eye having an upper eyelid and a lower eyelid, wherein the first electrode substrate is configured to locate the one or more electrodes at a front of the first eye of the patient, either directly on a cornea of the first eye or around the first eye of the patient;
a pupil-measurement device operatively coupled to the controller and configured to measure an aperture of a pupil of the first eye during delivery of the electrical-stimulation therapy;
a first return electrode configured to be placed in a return location on the patient that provides a return path for the electrical stimulation signals applied by the one or more stimulation electrodes such that an electrical current is formed between at least one of the one or more stimulation electrodes and the first return electrode; and
a stimulation controller operatively coupled to the one or more stimulation electrodes, a plurality of sensor electrodes, and the first return electrode, wherein the stimulation controller is configured to control the electrical current formed between the at least one of the one or more stimulation electrodes and the first return electrode such that the electrical current passes through a retina of the first eye of the patient and delivers the electrical-stimulation therapy.

14. The system of claim 13, wherein:
a) the stimulation controller is further configured to:
set a current level of the electrical-stimulation therapy at a first value during a first treatment session, wherein the first value of the current level is below a stimulation threshold of the first eye,
increase the current level from the first value during the first treatment session, and
determine a characteristic of the electrical-stimulation therapy during the first treatment session based at least in part on the measured aperture of the pupil of the first eye, wherein the characteristic includes a first indication that phosphenes are visible to the patient in the first eye during the first treatment session, and wherein the increase of the current level from the first value is stopped at a second value when the first indication is determined, or
b) the pupil-measurement device includes an infrared measurement device configured to measure infrared light emitted by the first eye.

15. The system of claim 13, wherein the first electrode substrate is configured to locate the one or more electrodes on an outer surface of at least one of the upper eyelid and the lower eyelid of the first eye of the patient.
